# EUROPEAN PATENT APPLICATION

(11) **EP 2 267 030 A1**
(43) Date of publication of application: **29.12.2010**
(21) Application number: 10009640.3
(22) Date of filing: 25.08.2006
(51) Int. Cl.: C07K 16/28, C07K 16/46, A61K 39/395

(54) **Devices, compositions and methods for the protection and repair of cells and tissues**

(30) Priority: 25.08.2005 US 711885 P
(62) Divisional of application: 06813768.6
(71) Applicant: Repair Technologies, Inc., Palo Alto, CA 94303 (US)
(72) Inventor: Slauter, Richard, San Jose CA 95120 (US); Moseley, Annemarie, B., Palo Alto CA 94303 (US); Lucero-Rajaram, Tamara, Los Altos CA 94024 (US)
(74) Representative: Grünecker, Kinkeldey, Stockmair & Schwanhäusser Anwaltssozietät

(57) **Abstract**

The present invention relates to compositions that reduce the extent of cellular and tissue damage in response to injury or disease. Methods of using the compositions to repair reperfusion injury, and structural tissue damage due to trauma, disease or aging are provided. Also provided are methods of using the compositions to reduce damage to transplanted tissue and organs. The compositions may also be used in oncology applications.

## Description

### BACKGROUND OF THE INVENTION

Many tissues and organs possess some repair capacity. The general process for tissue repair comprises three phases: inflammation, proliferation, and remodeling. Such endogenous repair mechanisms are commonly observed in the case of minor injuries to the skin. Examples of less well understood responses to injury include the regeneration of liver after resection and the reported recruitment of cells to the heart after myocardial infarction.

However, trauma or disease can overwhelm an organism's endogenous repair mechanisms and lead to the replacement of cells and tissues with scar or other nonfunctional tissue. In the case of ischemic injuries, such as stroke and myocardial infarction, cells in the ischemic zone that survived the initial attack are often at risk in the post-ischemic period. In fact, the restoration of blood flow to an area of ischemic damage itself leads to reperfusion injury. The net result of cellular death is a functional deficit.

Another example of inadequate natural tissue repair is that resulting from damage to the structural hyaline articular cartilage or the meniscus of the knee joint. Mesenchymal stem cells or marrow stromal cells, which give rise to the bone and cartilage lineages, have been shown, in osteochondral injury, to originate from the bone marrow near the site of injury and migrate into the site of injury. These cells are rare cells (frequency of 0.01% of the marrow mononuclear cells) and have been shown to bind to damaged cartilage extracellular matrix. Treatment of cartilage with structural matrix alone has resulted in poor repair, with few chondrocytes and an abundance of fibrous tissue being present at the site. Introduction of cartilage growth supportive agents, such as hyaluronic acid, have not demonstrated improvement in defect healing, and have provided only limited symptomatic relief. Introduction of differentiated chondrocytes did not result in significant repair in clinical trials, and animal trials using stem or progenitor cells without a structural matrix did not result in cartilage repair. Only an ex vivo differentiated hyaline cartilage tissue engineered construct which required full surgical implantation, and was composed of dedifferentiated cells, matrix and growth factor, has demonstrated any significant repair in animal studies.

In the case of organ failure, organ transplants represent a therapeutic final option for some patients. However, the number of suitable organs, e.g. hearts, kidneys, lungs and pancreases, is insufficient to meet demand. When an organ is available, the transplant procedure is expensive and the risk of morbidity and mortality is high, both in the acute and long term period after surgery. Generally, long term immunosuppression is required. Immunosuppressive drugs are expensive and are rarely completely effective, even when patients are fully compliant. Long-term immunosuppression can lead to health complications ranging from infection to increased susceptibility to some forms of cancer.

Some tissue and organ deficits have no viable transplant therapy. For example, researchers believe the heart has little or no endogenous repair capacity. Similarly, the brain is thought to have little or no endogenous repair capacity, insofar as damage to neural tissue is concerned.

Recently, a number of researchers have begun to examine stem cells as potential therapeutics in the emerging field of regenerative medicine. These researchers hope to obtain a sufficient number of biopharmaceutical-grade stem cells for administration to a patient Two broad types of stem cells are presently recognized: embryonic stem cells (ESCs) and adult stem cells (ASCs). Both cell types are characterized by their ability to proliferate extensively without differentiation and to send daughter cells down certain differentiation pathways, resulting in terminally differentiated cells of various lineages.

Some of the stem cells that have been isolated ex vivo and characterized according to phenotype and function include: ESCs (U.S. Pat. No. 5,843,780); bone marrow stromal cells (BMSC) or mesenchymal stem cells (Pittenger et al., 1999, Science 284(5411):143-7; U.S. Pat. No. 5,486,359); adipose tissue derived stromal cells (ADAS; U.S. Pat. No. 6,777,231); hematopoietic stem cells (HSCs; U.S. Pat. Nos. 4,965,204 and 5,061,620; neural stem cells (NSCs; U.S. Pat. No. 5,750,376); liver stem cells (U.S. Pat. No. 6,069,005); multipotent adult stem cells (MAPC; U.S. Pat. Publication No. 20040107453); retinal stem cells (U.S. Pat. No. 6,117,675); and pancreatic stem cells (U.S. Pat. No.6,866,843).

Progenitor cells are cells that, in terms of differentiation, reside between stem cells and terminally differentiated cells. These progenitor cells may retain substantial proliferative capacity, but are restricted in the lineages into which they may differentiate.

Both stem and progenitor cells are thought to differentiate into new tissue, enable recruitment of additional stem or progenitor cells, and/or secrete a combination of molecules enabling endogenous repair events. These cells are present in the blood and most tissues, and are mobilized to home to the injured site by acute injury signals, such as acute inflammatory cytokines. Tumor necrosis factor (TNF) is one such inflammatory cytokine. Prior studies have demonstrated that only rare numbers of these stem or progenitor cells can be found in the healing tissue, often in insufficient number to contribute to complete appropriate repair or tissue healing. Instead, the defect is replaced by scar tissue rather than normal tissue. In a similar manner, the administration of exogenous ex-vivo-expanded stem or progenitor cells has, in certain cases, demonstrated improved healing, but again, usually only a limited number of the cells are found in the area of injury.

Where inadequate repair results, the paucity of these cells at the site of injury may be due to a number of factors. It is possible that the injury signals are inadequate to direct these cells to the local site of injury. It is also possible that these cells do localize to the injured area, but do not remain there due to an inadequate environment in terms of either matrix or cell signaling factors. It is further possible that the few numbers of these cells that localize to the site cannot compete with the larger number of incoming fibroblastic scar-forming cells. Regardless of etiology, it is evident that the body's own mechanisms for attracting and maintaining repair cells is often not optimal for effective repair.

Exogenous BMSC administration has been reported to demonstrate improvements in the stroked rat (Chopp et al., 2002, Lancet Neurol. (2):92-100), in the pig following induced myocardial infarction (Amado et al, 2005, Proc Natl Acad Sci USA 102(32):11474-9) and in the meniscal injury osteoarthritis goat model (U.S. Pat. Publication No. 20020005205). However, these ex vivo approaches to the preparation of repair cells all suffer numerous shortcomings. Cell separation instruments can be enormously expensive and technically challenging to operate. Cell culture requires a substantial facility, including incubators, laminar flow hoods, cell washers and the like. Any ex vivo manipulation of cells raises the possibility not only of the introduction of artifacts of cell culture, but also of bacterial or viral contamination. The time required for isolation and culture, in many cases being weeks or months, makes many acute "off-the-shelf" therapies impossible. Preparation of cellular products for long-term storage is laborious, even when possible, and frequently calls for tedious cryopreservation, thawing and washing steps. Cells and tissues intended for a recipient other than the donor often necessitate immunosuppression to preclude graft rejection or graft vs host disease. The regulatory pathway is complex and involves substantial product characterization, potency and identity assays.

Thus, there is a long-felt need for tissue repair, including structural tissue repair, and for reduction of the severity of reperfusion injury in individuals subject to same. The present invention addresses this need.

### SUMMARY OF THE INVENTION

The present invention relates to a tissue repair composition comprising a first cell binding moiety that preferentially binds a damaged or defective cell, a second cell binding moiety that preferentially binds a repair cell, wherein the first and second cell binding moieties are linked. In an embodiment, the first and second cell binding moieties are linked to a common substrate. In an embodiment, the common substrate is avidin and at least one of the cell binding moieties is biotinylated. In other embodiments, the common substrate comprises a polymer or a hydrogel.

In an embodiment, the first cell binding moiety preferentially binds a cell surface molecule selected from the group comprising ICAM-1, N-CAM, a selectin, a cadherin, endothelin, CD44, V-CAM, P-CAM, collagen, fibronectin, hyaluronic acid, a proteoglycan, TGF-beta receptor and combinations thereof.

In an embodiment, the repair cell is selected from the group comprising an embryonic stem cell, a multipotent adult stem cell and a stromal cell. In an embodiment, the second cell binding moiety preferentially binds a cell surface molecule selected from the group comprising CD44, CD45, CD90, CD105, CD133, CD34, CD33, CD38, CD105, CD106, nestin and combinations thereof.

In an embodiment, the tissue repair composition further comprises a signaling factor linked to said first and second cell binding moieties. In an embodiment, the signaling factor is selected from the group consisting of EGF, VEGF, NGF, FGF, EPO, G-CSF, GM-CSF, PDGF, IGF-1, stem cell factor, stromal derived factor (SDF-1), MMP inhibitors, LIF, flt-1 ligand and combinations thereof.

The invention further provides a tissue repair composition comprising a first cell binding moiety that preferentially binds ICAM-1 and a second cell binding moiety that preferentially binds CD90, wherein the first and second cell binding moieties are linked. In an embodiment, the first and second cell binding moieties are linked to a common substrate. In an embodiment, the common substrate is avidin and at least one of the cell binding moieties is biotinylated. In an embodiment, the at least one of the cell binding moieties is an antibody. In an embodiment, the composition comprises a signaling factor, wherein said signaling factor is selected from the group consisting of EGF, VEGF, NGF, FGF, EPO, G-CSF, PDGF, IGF-1, stem cell factor, stromal derived factor, MMP inhibitors, LIF and combinations thereof, and wherein the signaling factor is linked to the first and second cell binding moieties.

The invention also provides a pharmaceutical composition comprising a tissue repair composition of the invention and a pharmaceutically acceptable excipient. In an embodiment, the tissue repair composition is soluble. In another embodiment, the tissue repair composition is bound to a matrix.

The invention further provides methods of using the composition of the invention. The methods are preferably used with humans.

A method of improving repair of injured tissue comprises contacting injured tissue with a therapeutically effective amount of a tissue repair composition of the invention.

A method of treating a mammal that has suffered an ischemic injury comprises administering a therapeutically effective amount of a tissue repair composition of the invention to the mammal.

A method of reducing tissue damage in a mammal that has suffered a stroke comprises administering a therapeutically effective amount of a tissue repair composition of the invention to the mammal.

A method of enhancing tissue repair in a mammal that has suffered a myocardial infarction comprises administering a therapeutically effective amount of a tissue repair composition of the invention to the mammal.

A method of enhancing tissue repair in a mammal that has suffered a spinal cord injury comprises administering a therapeutically effective amount of a tissue repair composition of the invention to the mammal.

A method of diminishing the severity of reperfusion injury in a human, comprises administering a therapeutically effective amount of a tissue repair composition of the invention to the human. In an embodiment, the human has received an organ transplant. In an embodiment, the organ for the organ transplant is chosen from the group comprising kidney, heart, lung, liver, pancreas, blood vessel, retina, skin and combinations thereof. In an embodiment, the reperfusion injury occurs upon reperfusion after vascular stenting, upon reperfusion after vascular angioplasty, upon reperfusion after thrombolytic therapy, upon reperfusion after coronary artery bypass surgery, or upon reperfusion after intestinal surgery.

A method of diminishing the severity of reperfusion injury to an organ intended for transplant comprises contacting the organ with a therapeutically effective amount of a tissue repair composition of the invention.

A method of enhancing tissue repair in damaged cartilage in a human comprises administering a therapeutically effective amount of a tissue repair composition of the invention to the human.

A method of enhancing tissue repair in damaged skin in a human comprises administering a therapeutically effective amount of a tissue repair composition of the invention to the human.

A method of treating renal damage in a human comprises administering a therapeutically effective amount of a tissue repair composition to the invention to the human.

In another embodiment, the use of a composition for the preparation of a medicament useful in any of the disclosed therapeutic methods is provided.

### BRIEF DESCRIPTION OF THE DRAWINGS

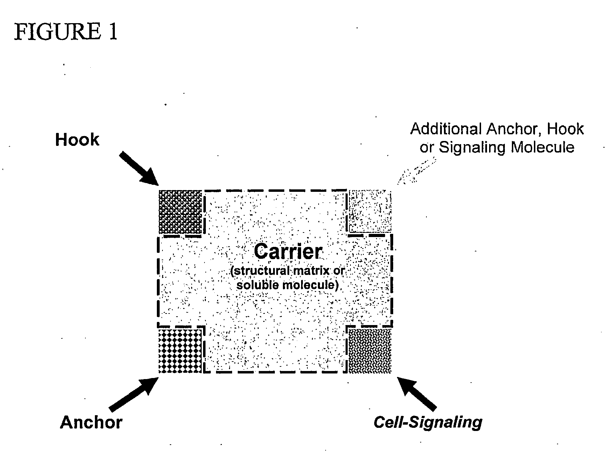

Figure 1 depicts a schematic of an embodiment of a repair composition. The compositions of the invention should not be construed as limited to the relative positioning of the various components depicted in the schematic.
Figure 2 depicts a representative histology section from control animal 2 at the time of sacrifice (day 6 after stroke and reperfusion). This section shows evidence of infarction and necrosis consistent with ischemic stroke and reperfusion injury.
Figure 3 depicts a representative histology section from treated animal 4 at the time of sacrifice (day 6 after stroke and reperfusion). There is no detectable histomorphologic evidence of infarction.
Figure 4 depicts an image of a representative histological brain section of an animal 6 days after being subjected to middle cerebral artery occlusion (MCAO) for two hours and then treated with saline.
Figure 5 depicts an image of a representative histological brain section of an animal 6 days after being subjected to MCAO for two hours and then treated with a repair composition comprising biotinylated anti-CD54 antibody and biotinylated anti-CD90 antibody bound to avidin.
Figure 6 depicts a graph of neurological outcomes at day 1 or day 6 for individual rats after being subjected to MCAO and treated with either saline (control), repair composition 1 (indicated by •) or repair composition 2.
Figure 7 depicts a graph of the composite data on neurological outcomes at day 1 or day 6 of rats subjected to MCAO and treated with either saline (control) or a repair composition (treated).
Figure 8 depicts a bar graph of data from a renal artery transient stenosis rat model subject to treatment with saline (control), Repair Composition 1 or Repair Composition 2.
Figure 9 depicts an image of a representative kidney section of a rat subjected to renal artery transient stenosis and treated with saline (control).
Figure 10 depicts an image of a representative kidney section of a rat subjected to renal artery transient stenosis and treated with Repair Composition 2.
Figure 11 depicts an image of a representative kidney section of a rat subjected to renal artery transient stenosis and treated with Repair Composition 3.
Figure 12 depicts a fluorescent image of DiI-labeled lymphocytes incubated with an aortic section treated with PBS.
Figure 13 depicts a fluorescent image of DiI-labeled lymphocytes incubated with an aortic section treated with a repair composition comprising anti-CD61 and anti-CD45 antibodies bound to avidin.
Figure 14 depicts a fluorescent image of DiI-labeled lymphocytes incubated with an aortic section treated with a repair composition comprising anti-CD106 and anti-CD45 antibodies bound to avidin.
Figure 15 depicts a fluorescent image of sectioned cardiac tissue with exposed left anterior descending (LAD) artery. The cardiac artery was ligated, then reperfused and a repair composition comprising anti-CD54 and anti-CD 90 antibodies was introduced into the LAD artery.
Figure 16 depicts a fluorescent image of femoral artery. The femoral artery was subjected to occlusion and reperfusion, then a repair composition comprising anti-CD54 and anti-CD 90 antibodies was introduced into the femoral artery. (40X magnification)
Figure 17 depicts a table of individual clinical hematology data for mice on Day 3 of a toxicity study of Repair Composition 2.
Figure 18 depicts a table of individual clinical chemistry data for mice on Day 3 of a toxicity study of Repair Composition 2.
Figure 19 depicts a table of individual clinical hematology data for mice on Day 7 of a toxicity study of Repair Composition 2.
Figure 20 depicts a table of individual clinical chemistry data for mice on Day 7 of a toxicity study of Repair Composition 2.
Figure 21 depicts a table of individual clinical hematology data for mice on Day 10 of a toxicity study of Repair Composition 2.
Figure 22 depicts a table of individual clinical chemistry data for mice on Day 10 of a toxicity study of Repair Composition 2.

### DETAILED DESCRIPTION

The present invention relates to the discovery of devices, compositions and methods that reduce the extent of cellular and tissue damage in response to injury or disease. The inventive compositions localize repair cells at a site of injury. One form of injury addressed by the present invention is structural tissue damage due to trauma, disease or aging. Another form of injury addressed is reperfusion injury.

### Definitions

As used herein, each of the following terms has the meaning associated with it in this section.

The articles "a" and "an" are used herein to refer to at least one of the object of the article. Thus, "an element" means one element or more than one element.

The term "about" will be understood by persons of ordinary skill in the art and will vary to some extent on the context in which it is used.

"Allogeneic" refers to material derived from a different animal of the same species.

"Anchor" refers to a cell binding moiety that preferentially binds a damaged or defective cell. An "anchor target" refers to a molecule on a damaged or defective cell to which the cell binding moiety preferentially binds.

The term "antibody," as used herein, refers to an immunoglobulin molecule which is able to specifically bind to a specific epitope on an antigen. Antibodies can be intact immunoglobulins derived from natural sources or from recombinant sources and can be immunoreactive portions of intact immunoglobulins. Antibodies are typically tetramers of immunoglobulin molecules. The antibodies in the present invention may exist in a variety of forms including, for example, polyclonal antibodies, monoclonal antibodies, intracellular antibodies ("intrabodies"), Fv, Fab and F(ab)₂, as well as single chain antibodies (scFv), camelid antibodies and humanized antibodies (Harlow et al., 1999, Using Antibodies: A Laboratory Manual, Cold Spring Harbor Laboratory Press, NY; Harlow et al., 1989, Antibodies: A Laboratory Manual, Cold Spring Harbor, New York; Houston et al., 1988, Proc. Natl. Acad. Sci. USA 85:5879-5883; Bird et al., 1988, Science 242:423-426).

By the term "synthetic antibody" as used herein, is meant an antibody which is generated using recombinant DNA technology, such as, for example, an antibody expressed by a bacteriophage as described herein. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using synthetic DNA or amino acid sequence technology which is available and well known in the art.

As used herein, "aptamer" refers to a small molecule that can bind specifically to another molecule. Aptamers are typically either polynucleotide- or peptidebased molecules. A polynucleotidal aptamer is a DNA or RNA molecule, usually comprising several strands of nucleic acids, that adopt highly specific three-dimensional conformation designed to have appropriate binding affinities and specificities towards specific target molecules, such as peptides, proteins, drugs, vitamins, among other organic and inorganic molecules. Such polynucleotidal aptamers can be selected from a vast population of random sequences through the use of systematic evolution of ligands by exponential enrichment. A peptide aptamer is typically a loop of about 10 to about 20 amino acids attached to a protein scaffold that bind to specific ligands. Peptide aptamers may be identified and isolated from combinatorial libraries, using methods such as the yeast two-hybrid system.

"Autologous" means material derived from the same animal to which it is later administered.

A "biocompatible material" refers herein to any material, which, when administered to or implanted in a mammal, does not provoke an adverse response in the mammal. A biocompatible material, when introduced into an individual, is not toxic or injurious to that individual, nor does it induce immunological rejection of the material in the mammal.

A "cell binding moiety" is a molecule which is capable of binding specifically to a receptor, ligand or other cell surface component on a cell membrane.

As used herein, "common substrate" refers to a molecule or material capable of binding at least one hook moiety and at least one anchor moiety. Binding to a common substrate may be covalent or noncovalent.

"Damaged or defective cell" refers to a cell that is injured or otherwise under biological, traumatic or oxidative stress.

As used herein "endogenous" refers to any material produced within or originating inside an organism.

"Exogenous" refers to any material introduced into or produced outside an organism.

As used herein, "expression cassette" refers to a nucleic acid molecule comprising a coding sequence operably linked to promoter/regulatory sequences necessary for transcription and translation of the coding sequence.

"Hook" refers to a cell binding moiety that preferentially binds a repair cell (RC). A "hook target" refers to a molecule on a repair cell to which the cell binding moiety preferentially binds.

As used herein, an "instructional material" includes a publication, a recording, a diagram, or any other medium of expression which can be used to communicate the usefulness of the composition of the invention for its designated use. The instructional material of the kit of the invention may, for example, be affixed to a container which contains the composition or be shipped together with a container which contains the composition. Alternatively, the instructional material may be shipped separately from the container with the intention that the instructional material and the composition be used cooperatively by the recipient

As used herein, "in vitro" and "ex vivo" are used to refer to conditions outside the body of a living organism. Thus, in vitro culturing and ex vivo culturing both refer to culturing, maintaining and/or manipulation of living cells, tissues or organs outside the body of a living organism. Ex vivo generally implies that the cells, tissues or organs that are cultured, maintained and/or manipulated outside of the body are returned to the body. In contrast, cells, tissues or organs cultured, maintained or manipulated in vitro are generally not intended to be returned to the body of an organism.

"Linked" refers to noncovalent or covalent bonding between two or more molecules. Linking may be direct or indirect.

As used herein, "matrix" refers to any material to which a plurality of a hook moiety and a plurality of an anchor moiety of a repair composition may be bound, covalently or noncovalently. As used herein, "plurality" refers to at least about 3, preferably at least about 100 and more preferably, at least about 1000. For example, a hydrogel comprising a plurality of a hook moiety and a plurality of an anchor moiety distributed throughout the hydrogel and without specific structural relationship to each other is a matrix. Another example of a matrix is a molecule, such as dextran, permitting multiple attachment sites for the plurality of moieties. A matrix may provide structural support such as meshes or fabrics. A matrix material may be self-assembling from peptide or polymer subunits, or nanomer starting materials. A matrix may localize a molecule to a fixed point in a tissue. A matrix may localize a molecule to a fixed point on the matrix but the matrix itself may be free to move, for instance, in a solution. Non-limiting examples of materials useful as a matrix in the invention include polyglactin 910 (Vicryl^{®}), dextran, polyglycolic acid (PGLA), decellularized small intestinal lining (SIS^{®}), woven hyaluronic acid mesh (HYAFF^{®}), and collagen matrix.

"Naturally-occurring" as applied to an object refers to the fact that the object can be found in nature. For example, a polypeptide or polynucleotide sequence that is present in an organism (including viruses) that can be isolated from a source in nature and which has not been intentionally modified by man is naturally-occurring.

"Pharmaceutically acceptable carrier" refers herein to compositions suitable for delivering the inventive composition in humans without excessive toxicity or other complications while maintaining the biological activity of the composition. Protein stabilizing excipients, such as mannitol, sucrose, polysorbate-80 and phosphate buffers, are typically found in such carriers, although the carriers should not be construed as being limited only to these compounds.

A "polynucleotide" means a single strand or parallel and anti-parallel strands of a nucleic acid. Thus, a polynucleotide may be either a single-stranded or a double-stranded nucleic acid.

The term "nucleic acid" typically refers to large polynucleotides.

The term "oligonucleotide" typically refers to short polynucleotides, generally no greater than about 50 nucleotides. It will be understood that when a nucleotide' sequence is represented by a DNA sequence (i.e., A, T, G, C), this also includes an RNA sequence (i.e., A, U, G, C) in which "U" replaces "T."

Conventional notation is used herein to describe polynucleotide sequences: the left-hand end of a single-stranded polynucleotide sequence is the 5'-end; the left-hand direction of a double-stranded polynucleotide sequence is referred to as the 5'-direction.

The direction of 5' to 3' addition of nucleotides to nascent RNA transcripts is referred to as the transcription direction. The DNA strand having the same sequence as an mRNA is referred to as the "coding strand"; sequences on the DNA strand which are located 5' to a reference point on the DNA are referred to as "upstream sequences"; sequences on the DNA strand which are 3' to a reference point on the DNA are referred to as "downstream sequences."

By describing two polynucleotides as "operably linked" is meant that a single-stranded or double-stranded nucleic acid moiety comprises the two polynucleotides arranged within the nucleic acid moiety in such a manner that at least one of the two polynucleotides is able to exert a physiological effect by which it is characterized upon the other. By way of example, a promoter operably linked to the coding region of a gene is able to promote transcription of the coding region.

As used herein, a "peptidomimetic" is a compound containing non-peptidic structural elements that is capable of mimicking the biological action of a parent peptide. A peptidomimetic may or may not comprise peptide bonds.

"Polypeptide" refers to a polymer composed of amino acid residues, related naturally occurring structural variants, and synthetic non-naturally occurring analogs thereof linked via peptide bonds. Synthetic polypeptides can be synthesized, for example, using an automated polypeptide synthesizer.

The term "protein" typically refers to large polypeptides.

The term "peptide" typically refers to short polypeptides.

Conventional notation is used herein to portray polypeptide sequences: the left-hand end of a polypeptide sequence is the amino-terminus; the right-hand end of a polypeptide sequence is the carboxyl-terminus.

"Preferentially bind" as used herein refers to the higher affinity of a binding molecule for a target molecule compared to the binding molecule's affinity for non-target molecule. A binding molecule that preferentially binds a target molecule does not substantially recognize or bind non-target molecules.

As used herein, the term "promoter/regulatory sequence" means a nucleic acid sequence which is required for expression of a gene product operably linked to the promoter/regulator sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

A "constitutive promoter" is a promoter which drives expression of a gene to which it is operably linked, in a constant manner in a cell. By way of example, promoters which drive expression of cellular housekeeping genes are considered to be constitutive promoters.

An "inducible" promoter is a nucleotide sequence which, when operably linked with a polynucleotide which encodes or specifies a gene product, causes the gene product to be produced in a living cell substantially only when an inducer which corresponds to the promoter is present in the cell.

A "tissue-specific" promoter is a nucleotide sequence which, when operably linked with a polynucleotide which encodes or specifies a gene product, causes the gene product to be produced in a living cell substantially only if the cell is a cell of the tissue type corresponding to the promoter.

"Repair cell" (RC) refers herein to a cell that serves to aid, stimulate or otherwise enable tissue repair. For example, a repair cell can be a stem cell or a committed progenitor cell that is capable of participating in the repair and/or protection of cells, tissues and organs. Committed progenitor cells may or may not be activated in their role as repair cells. Non-limiting examples of RCs are: embryonic stem cells (ESCs), bone marrow stromal cells (BMSC) or mesenchymal stem cells, adipose tissue derived stromal cells, hematopoietic stem cells (HSCs), neural stem cells (NSCs), liver stem cells, multipotent adult stem cells, retinal stem cells and pancreatic stem cells. An RC may be endogenous to an organism, or it may be provided exogenously to an organism.

"Signaling factor" as used herein refers to a substance that either stimulates proliferation, division and/or maturation of cells that contribute positively to tissue repair or inhibits normal cellular activity that causes, propagates or perpetuates tissue injury. For instance, a composition administered to a subject immediately after ischemic injury, for instance from a stroke, may comprise a signaling factor that inhibits leukocyte activity, which contributes to reperfusion injury. A composition administered a week later to the subject may comprise a signaling factor that stimulates a repair cell, such as a BMSC, in a post-stroke nerve injury to contribute to the repair of the tissue injury.

The term "substantially pure" describes a compound, e.g., a protein or polypeptide which has been separated from components which naturally accompany it. Typically, a compound is substantially pure when at least 10%, more preferably at least 20%, more preferably at least 50%, more preferably at least 60%, more preferably at least 75%, more preferably at least 90%, and most preferably at least 99% of the total material (by volume, by wet or dry weight, or by mole percent or mole fraction) in a sample is the compound of interest. Purity can be measured by any appropriate method, e.g., in the case of polypeptides by column chromatography, gel electrophoresis or HPLC analysis. A compound, e.g., a protein, is also substantially purified when it is essentially free of naturally associated components or when it is separated from the native contaminants which accompany it in its natural state.

A host cell that comprises a recombinant polynucleotide is referred to as a "recombinant host cell." A gene which is expressed in a recombinant host cell wherein the gene comprises a recombinant polynucleotide, produces a "recombinant polypeptide."

"Recombinant polynucleotide" refers to a polynucleotide having sequences that are not naturally joined together. An amplified or assembled recombinant polynucleotide may be included in a suitable vector, and the vector can be used to transform a suitable host cell.

A recombinant polynucleotide may serve a non-coding function (e.g., promoter, origin of replication, ribosome-binding site, etc.) as well.

A "recombinant polypeptide" is one which is produced upon expression of a recombinant polynucleotide.

A "tissue repair composition" as used herein refers to a composition that treats injured tissue, prevents further damage to injured tissue, or treats tissue contaminated with cancer cells, for instance, metastatic cancer cells, which, if left untreated, would lead to a tissue injury.

"Therapeutically effective amount" means a nontoxic amount of the inventive composition sufficient to provide a beneficial effect to the subject to which the composition is introduced.

"Treating" as used herein means ameliorating the effects of, or delaying, halting or reversing the progress of, a condition. The word encompasses reducing the severity of a symptom of a condition and/or the frequency of a symptom of a medical condition.

As used herein, a phrase describing a change in injury or repair refers to a change relative to the same injury or repair in the absence of administration of a repair composition. Such phrases include "diminish the severity of a reperfusion injury", "imporiving repair of injured tissue", "enhance tissue repair", and "reducing tissue damage." Assessing the severity of a reperfusion injury encompasses length of time to injury after injury cause, extent of injury, duration of injury and length of time to repair injury. Diminishing severity, therefore, refers to at least one of slowing the time to injury, reducing the extent of injury, and reducing the duration of the injury, as measured by clinically significant parameters known in the art. Similarly, reducing tissue damage refers to at least one of slowing the time to injury after injury cause, reducing the extent of tissue damage, reducing the severity of tissue damage, reducing the duration of tissue damage and decreasing the length of time to repair, as measured by clinically significant parameters known in the art. Improving repair of injured tissue and enhancing tissue repair refer to at least one of increasing the rate of repair, decreasing the extent of tissue injury, and decreasing the severity of injury, as measured by clinically significant parameters known in the art.

The techniques and procedures for recombinant manipulations, including nucleic acid and peptide synthesis, are generally performed according to conventional methods in the art and various general references (e.g., Sambrook et al, 2001, Molecular Cloning, A Laboratory Approach, Cold Spring Harbor Laboratory Press, Cold Spring Harbor, NY; Ausubel et al., eds, 2005, Current Protocols in Molecular Biology, John Wiley & Sons, New York, NY; and Gerhardt et al., eds., 1994, Methods for General and Molecular Bacteriology, American Society for Microbiology, Washington, DC), which are provided throughout this document.

It is understood that any and all whole or partial integers between any ranges set forth herein are included herein.

### Detailed Description

The invention is a composition that, in one embodiment, is able to bind to injured or damaged cells or tissue and recruit repair cells to the site, thereby reducing the injury or damage. In some embodiments, the composition also enhances healing or recovery from injury. The composition of the invention is therefore useful in therapeutic methods of reducing damage, improving repair of injured or damaged tissue, diminishing the severity and extent of reperfusion injury, enhancing tissue repair in damaged cartilage and enhancing tissue repair of damaged skin, among others. In other embodiments, the composition of the invention is designed to recruit repair cells to the site of injury or damage. In other embodiments, the composition of the invention is designed to bring repair molecules, such as cytokines, hormones, growth factors and proteoglycans, to cells at an injured site. Many other applications of the composition of the invention will be apparent to the skilled artisan in view of the instant disclosure.

Compositions of the present invention comprise a hook molecule linked directly, or indirectly via a common substrate, to an anchor molecule. The hook molecule is a moiety that preferentially binds to a repair cell. The anchor molecule is a moiety that binds preferentially to a damaged or defective cell. Without being bound by theory, it is believed that administration of the inventive composition increases the likelihood that needed repair cells will bind to injured tissues and that the residency time of the repair cells will be increased at the site of injury. However, the mechanism by which a composition of the invention contributes to the repair of injured tissues should not be construed as a limitation on the composition or methods of using it. For instance, if the hook molecule of a composition of the invention is found to bind something other than a repair cell while contributing to the repair of an injured tissue, the composition and its use is still encompassed by the invention.

In some embodiments, the composition further comprises a signaling factor which may contribute to repair of injured tissues by cellular modification or stimulation of other cells. In some embodiments, the composition further comprises a repair cell recognized by the hook molecule. Thus, when this composition is administered to a subject, the repair cell is provided exogenously. However, the invention is not limited to administering an exogenous repair cell. Without being bound by theory, it is believed that composition of the invention will bind and retain endogenous repair cells at the site of injury, thereby increasing the effective local concentration of such rare endogenous repair cells.

### Components Useful in Composition

The hook moiety, anchor moiety and signaling factor may comprise small molecules, peptides, recombinant proteins, antibodies, including antibody fragments, peptidomimetics or aptamers. Preferably, the hook and anchor are antibodies.

Molecules that bind preferentially to repair cells or to damaged cells are well known to those of ordinary skill in the art and are copiously published in the literature. For instance, neural stem and progenitor cells, adult stem and progenitors cells arising from the blood (CD34+Thy-1+) cells, as well as adult mesenchymal stem and progenitor cells, adipose-derived stromal cells and marrow stromal cells express CD90 (Thy-1). Thus, in a preferred embodiment, the composition comprises a moiety that preferentially binds to CD90. Preferably, the moiety is an antibody against CD90. Cell surface markers for other stem and progenitor cells are known. For instance, cell surface markers specific for HSCs include CD34, CD45, CD41, and ABCG2. Other markers for HSCs and various hematopoietic committed lineages include CD2, CD3, CD4, CD5, CD6, CD7, CD8, CD13, CD14, CD15, CD19, CD20, CD32, CD51, CD52, CD53, CD56, CD57, CD60, CD61, CD62L, CD65, CD72, CD73, CD81, CD82, CD83, CD99, CD100, CD117, TCR, and HLA-DR. Markers for adipose-derived stromal cells include HLA-ABC and CD29 (integrin β1), CD49e (integrin α5/VLA-5) and CD51 (integrin αV). Other markers include CD49b (integrin α2/VLA-2), CD49d (integrin α4/VLA-4), CD61 (integrin β3), CD138 (syndecan-1), and CD140a (PDGFR-β). Additional phenotypic markers continue to be disclosed for various repair cells or can be identified without undue experimentation.

Molecules, including but not limited to, antibodies, which preferentially bind to cell surface markers specific to repair cells or to damaged cells are well known in the art and are also readily identified without undue experimentation. See, for instance, U.S. Pat. Nos. 5,654,282, 5,632,991, 6,177,547, 5,827,670, 5,756,095, 5,565,550, 6,506,382, and 5,951,982, and EP 00528931.

It is known in the art that certain cell surface markers are expressed in response to cell and tissue injury. An example of an injured cell is a cell that has suffered a reperfusion injury, such as that caused when blood flow is restored to a tissue after a period of ischemia. Other such injuries include those due to trauma, aging, and disregulation of metabolic processes and controls. Molecules of the integrin family, such as ICAM-1, are known to be expressed in tissues and organs, such as brain and heart, after an ischemic injury. In particular, the expression of the integrin ICAM-1 is increased on injured cells. Thus, in a preferred embodiment, the composition comprises a moiety that preferentially binds to ICAM-1. Preferably, the moiety is an anti-ICAM-1 antibody. Without being bound by theory, a composition of the invention comprising a molecule that preferentially binds ICAM-1 will serve to bring a repair cell into contact with the site of an ischemic injury. Other cell surface molecules useful as the preferential target of the hook moiety of the composition include, but are not limited to, selectins, cadherins, alpha integrins, fibronectin, collagen, proteoglycans, N-CAM, V-CAM, endothelin, CD44 and endothelin.

In a preferred embodiment, the composition comprises an anchor moiety that preferentially binds to ICAM-1 and a hook moiety that preferentially binds to CD90 (Thy-1). In one aspect, the anchor moiety is an anti-ICAM-1 antibody and the hook moiety is an anti-CD90 antibody.

The composition of the invention optionally comprises a signaling factor that serves to contribute to injury repair. Signaling factors useful in the inventive composition include, but are not limited to, growth hormone, vascular endothelial growth factor (VEGF), transforming growth factor-beta (TGF-beta), erythropoietin (EPO), epidermal growth factor (EGF), c-kit ligand/stem cell factor, insulin, insulin-like growth factors, insulin like growth factor-1 (IGF-1), fibroblast growth factor (FGF), nerve growth factor (NGF), platelet derived growth factor (PDGF), bone morphogenetic protein (BMP), leukemia inhibitory factor (LIF), flt-1 ligand, brain derived neurotrophic factor (BDNF), interleukins such as but not limited to interleukin 3 (IL-3), interleukin 6 (IL-6), interleukin 7 (IL-7), and interleukin 13 (IL-13), stromal derived factor (SDF), stem cell factor (SCF), granulocyte colony stimulating factor (G-CSF), and matrix metallo-proteinase (MMP) inhibitors.

Armed with this disclosure and the teachings in the art, the skilled artisan can select a signaling factor useful for a given composition based on the injury to be treated. For instance, the art teaches that EGF stimulates growth and differentiation of neural progenitors and stem cells. EGF has also been implicated in angiogenesis and thus may improve revascularization. EGF also mitigates adverse effects of reactive oxygen metabolites following ischemia reperfusion injury. Accordingly, a composition intended to treat ischemic injury, such as that associated with stroke or renal vascular injury, is benefited by the addition of EGF.

In a preferred embodiment, the composition comprises an anchor moiety that preferentially binds to ICAM-1, a hook moiety that preferentially binds to CD90 (Thy-1) and EGF. In one aspect, the anchor moiety is an anti-ICAM-1 antibody and the hook moiety is an anti-CD90 antibody. The composition is useful for treating reperfusion injury, for instance, resulting from stroke.

Other suitable choices for anchor molecules in a composition for treating reperfusion injury include selectins, preferably P-selectin, CD44 and N-CAM. A preferred target for the hook moiety is CD90, which is present on RCs originating from the marrow, adipose tissue, neural progenitors and hematopoietic stem cells. Other targets for the hook moiety include CD44, CD105, CD133 and nestin. Optional cell signaling factors include EGF, which stimulates growth and differentiation of neural progenitors and stem cells; EGF also has an angiogenic effect. Other embodiments include FGF, PDGF, EPO, BDNF and VEGF.

For compositions useful for treating renal damage, including but not limited to, renal failure and acute tubular necrosis (ATN), suitable anchor targets include CD54, selectins, cadherins and endothelin. Suitable hook targets include CD90, CD105, CD133, CD106 and CD34. Signaling factors suitable for use in a composition for treating renal damage include, but are not limited to, EGF, FGF, PDGF, EPO, BMP and VEGF.

For compositions useful for treating injured connective tissues, such as cartilage and meniscus, suitable anchor targets include ICAM-1, hyaluronic acid, collagens, proteoglycans, fibronectins and the TGFbeta-receptor. Suitable hook moieties include anti-CD105, anti-CD44, anti-CD90, anti-CD133 and anti-CD34. Armed with the present disclosure, one of skill in the art may choose an RC of interest, such as osteochondral progenitors, chondrocytes, synovial cells, and BMSCs, to be targeted by the hook moiety. Cell signaling molecules such as TGFbeta, FGF, PDGF and matrix metallo-proteinase (MMP) inhibitors are optionally included in the construct as a signaling factor.

For compositions useful for treating traumatic skin injury, an anchor which targets molecules expressed after injury, such as an alpha integrin, ICAM-1, fibronectin, hyaluronic acid, collagen, or proteoglycans, is suitable. Suitable hook moieties include anti-CD44, anti-CD105, anti-CD90, anti-CD133 and anti-CD34.

For compositions useful for treating myocardial ischemic injuries, suitable anchor targets include ICAM-1, selectins, CD44 and V-CAM. Suitable hook target moieties include CD90, CD44, CD105, CD133 and CD106. A preferred optional cell signaling factor is VEGF. Other cell signaling factors include FGF, PDGF, EPO and EGF.

Compositions useful for treating spinal cord injuries have an anchor molecule that targets molecules that are upregulated after the injury. Such molecules include integrins, such as N-CAM, ICAM-1 and combinations of both. Other suitable targets include selectins, CD44 and cadherins. The preferred target for the hook molecule is CD90, which is present on RCs originating in the marrow, including neural progenitors and hematopoietic stem cells. CD90 is also present on ADAS cells originating in adipose tissue. Other hook targets include CD44, CD105, CD133 and nestin. Optional cell signaling factors include FGF, which stimulates growth and differentiation of neural progenitors and stem cells, and has angiogenic effects. Other suitable signaling factors include EGF, PDGF, EPO and VEGF. Compositions for spinal cord injury may comprise avidin to which the moieties are bound, as described elsewhere herein. For treatments where space filling is desirable, such as treatment of cysts, the moieties of the composition may be bound to a biocompatible mesh, such as Vicryl^{®}.

Compositions of the invention may be used to treat organs intended for donation. The organs may be treated while in the organ donor, as well as after their removal from the donor, during transplantation and subsequent to transplantation. The preferred anchor target for such compositions is ICAM-1. Preferably the anchor is a monoclonal antibody to ICAM-1. Other anchor targets include selectins, CD44 and V-CAM. The preferred target for the hook is CD90, which is present on hematopoietic stem and endothelial progenitor RCs originating in the marrow and on ADAS cells originating in adipose tissue. Other suitable hook targets include CD44, CD105 and CD133. Optional cell signaling factors include VEGF, FGF, PDGF, EPO and EGF.

The clinician may treat the recipient at the time of transplantation or anytime thereafter in the first month with repair composition local or distant to the site of organ transplantation. Upon clinical signs of rejection of the transplant, the physician may again administer the repair composition to the patient.

Compositions useful for cancer immunotherapy treatment comprise anchor molecule that targets tumor cell markers. Non-limiting examples of tumor cell markers include: MAGE 1, 2, & 3, MART-1/Melan-A, gp100, carcinoembryonic antigen (CEA), HER-2, mucins (i.e., MUC-1), prostate-specific antigen (PSA), prostatic acid phosphatase (PAP), hepatitis B (HBV), Epstein-Barr (EBV), human papilloma (HPV) p53, and glycosylate proteins. Anchor molecules useful in these compositions comprise a molecule that binds a cell of the immune system, such as a T cell. Targets on T cells include, but are not limited to, CD3, CD8, and CD4. Optional signaling factors useful in these compositions include cytokines, such as, but not limited to, IL-2, IL-4, IL-5, IL-13 and IL-18. The composition may further comprise molecules that bind to co-stimulatory molecules, such as, but not limited to, CDw137, B7-H2, CD275, CD28, CD40 (BP50), CD80 (B7-1), CD86 (B7-2), CD150 (SLAM), CD154 (CD40 Ligand), GITR Ligand, ICOS (CD278), and ICOSL (B7-H2, CD275).

Optionally, the composition of the invention may further comprise one or more moieties for additional therapeutic benefit. Such moieties may be antagonistic to ligands that induce an undesirable effect. Such moieties may bind to a receptor for a ligand, or may bind to the ligand itself, to prevent or reduce the effects of the ligand. For instance, it is useful to antagonize the effects of pro-inflammatory cytokines, such as TNF-α and IL-1, in the setting of tissue grafts, to reduce the initiation of graft injury. Alternatively, additional moieties may act as agonists, to induce desirable effects of ligands or may themselves be cytokines.

Table 1 provides exemplary human CD markers useful as hook and anchor targets, and optional signaling factors suitable for use in the composition of the invention and the target damaged cell or tissue for which such a composition is useful therapeutically. This list is not meant to be exhaustive and therefore, should not be construed as limiting the invention. Armed with this disclosure and the teachings in the art, the skilled artisan can select combinations of hook and anchor moieties to prepare a composition of the invention, such that the composition is useful for treating a particular injury of interest.

**Table 1**

| **Injury (tissue/cell)** | **Hook target** | **Anchor target** | **Optional Signaling Factor** |
|---|---|---|---|
| **Brain** | CD105, Nestin, CD90, CD133, CD34, CD44 | CD54, CD56, selectins such as CD62L and CD62P, cadherins such as CD144, endothelin, CD44 | EGF, FGF, PDGF, EPO, G-CSF, VEGF, BDNF |
| **Heart** | CD105, CD90, CD44, CD133, CD106, CD34 | CD54, selectins, cadherins, CD44, CD106 | VEGF, EGF, FGFb, PDGF, EPO |
| **Spinal cord** | CD105, Nestin, CD90, CD133, CD34, CD44 | CD54, CD56, selectins, cadherins, endothelin, CD44 | EGF, FGF, PDGF, EPO, VEGF |
| **Kidney** | CD105, CD90, CD133, CD106, CD34 | CD54, selectins, cadherins, endothelin | EGF, FGF, PDGF, EPO, BMP, VEGF |
| **Lung** | CD105, CD90, CD133, CD106, CD34, CD44 | CD54, CD31, selectins, cadherins, endothelin | VEGF, FGF, PDGF |
| **Pancreas** | CD105, CD90, CD133, CD106, CD34 | CD54, selectins, cadherins, endothelin | EGF, FGF, PDGF, EPO, HGF, IGF-1 |
| **Liver** | CD105, CD90, CD133, CD106, CD34, EP-CAM, CD117 | CD54, selectins, cadherins, endothelin | EGF, FGF, PDGF, IGF-1, HGF |
| **Retina** | CD105, CD90, CD133; CD106, CD34 | CD54, selectins, cadherins, endothelin | EGF, FGF, PDGF, EPO, G-CSF |
| **Skin** | CD105, CD90, CD133, CD34, CD44 | CD54, fibronectin, hyaluronic acid, collagen, proteoglycans, CD44, alpha integrins, selectins, cadherins, endothelin | EGF, FGF, PDGF, EPO |
| **Blood vessel** | CD105, CD90, CD133, CD106, CD34, CD31, CD33, von Willebrand Factor | CD54, CD31, selectins, cadherins, endothelin | VEGF, EGF, FGF, PDGF, EPO |
| **Cartilage** | CD105, CD90, CD133, CD106, CD34, CD44 | CD54, fibronectin, proteoglycans, collagen, TGF-beta receptor, CD44 | TGF-beta, FGF, PDGF, BMP, MMP inhibitors |
| **Bone marrow** | CD45, CD34, CD33, CD38, CD105 | CD45, CD34, CD33, CD38, CD105 | SDF-1, stem cell factor, GM-CSF, G-CSF, EPO, LIF, flt-1 ligand |

In some embodiments, the composition further comprises a repair cell recognized by the hook molecule. Thus, when this composition is administered to a subject, the repair cell is provided exogenously. Stem or progenitor cells that can mediate the repair process may be obtained by way of cell or tissue donation. The explant is then manipulated ex vivo to isolate the desired cells. Isolation is typically performed on the basis of surface markers, density, size and other physical characteristics. For instance, the unique cell surface markers on the repair cells of interest can be used to isolate a specific subpopulation of cells from a mixed population of cells. One of ordinary skill in the art will recognize that known colorimetric, fluorescent, immunochemical, polymerase chain reaction, chemical or radiochemical methods can readily ascertain the presence or absence of a lineage specific marker or enzymatic activity. When isolation is performed by way of extensive culturing, the growth characteristics of the cells may allow for enrichment or purification of the target cell type. For example, both bone marrow stromal cells (BMSCs) and adipose tissue derived stromal cells adhere to tissue culture plastic and grow in culture, permitting for numerical expansion. Some stem cells, such as HSCs, are difficult to expand in culture without undergoing terminal differentiation. Selection by affinity, cell sorting on flow cytometers and magnetic cell separation techniques are also known in the art.

In a preferred embodiment of the invention, the hook and anchor moieties are antibodies or antibody fragments. Preferably, the antibodies are from the same species as the intended recipient. For instance, antibodies for compositions intended for human recipients are preferably human antibodies or humanized antibodies. In a preferred embodiment, a composition of the invention comprises an antibody to ICAM-1 and an antibody to CD90. The composition optionally comprises EGF. In one aspect, the antibodies are bound to a common substrate. In another aspect, the antibodies are linked directly to each other.

The generation of polyclonal and monoclonal antibodies against a specific antigen is well known in the art. The generation of polyclonal antibodies is accomplished by inoculating the desired animal with the antigen and isolating antibodies which specifically bind the antigen therefrom.

Monoclonal antibodies directed against full length or peptide fragments of a protein or peptide may be prepared using any well known monoclonal antibody preparation procedures, such as those described, for example, in Harlow et al. (1988, In: Antibodies, A Laboratory Manual, Cold Spring Harbor, NY) and in Tuszynski et al. (1988, Blood, 72:109-115). Human monoclonal antibodies may be prepared by the method described in U.S. patent publication 2003/0224490. Quantities of the desired peptide may also be synthesized using chemical synthesis technology. Alternatively, DNA encoding the desired peptide may be cloned and expressed from an appropriate promoter sequence in cells suitable for the generation of large quantities of peptide. Monoclonal antibodies directed against the peptide are generated from mice immunized with the peptide using standard procedures as referenced herein.

DNA coding sequences encoding the cell surface markers of interest are well known in the art. For instance, the nucleotide sequence and amino acid sequence of human ICAM-1 are available as GenBank Accession Nos. NM_000201 and NP_000192, respectively, incorporated herein by reference in their entirety. The nucleotide sequence and amino acid sequence of human CD90 are available as GenBank Accession Nos. NM_006288 and NP_006279, respectively, incorporated herein by reference in their entirety.

Nucleic acid encoding the monoclonal antibody obtained using the procedures described herein may be cloned and sequenced using technology which is available in the art, and is described, for example, in Wright et al. (1992, Critical Rev. in Immunol. 12(3,4):125-168) and the references cited therein. Further, the antibody of the invention may be "humanized" using the technology described in Wright et al., (supra) and in the references cited therein, and in Gu et al. (1997, Thrombosis and Hematocyst 77(4):755-759).

To generate a phage antibody library, a cDNA library is first obtained from mRNA which is isolated from cells, e.g., the hybridoma, which express the desired protein to be expressed on the phage surface, e.g., the desired antibody. cDNA copies of the mRNA are produced using reverse transcriptase. cDNA which specifies immunoglobulin fragments are obtained by PCR and the resulting DNA is cloned into a suitable bacteriophage vector to generate a bacteriophage DNA library comprising DNA specifying immunoglobulin genes. The procedures for making a bacteriophage library comprising heterologous DNA are well known in the art and are described, for example, in Sambrook et al. (2001, Molecular Cloning: A Laboratory Manual, Cold Spring Harbor Laboratory Press, Cold Spring Harbor, NY).

Bacteriophage which encode the desired antibody, may be engineered such that the protein is displayed on the surface thereof in such a manner that it is available for binding to its corresponding binding protein, e.g., the antigen against which the antibody is directed. Thus, when bacteriophage which express a specific antibody are incubated in the presence of a cell which expresses the corresponding antigen, the bacteriophage will bind to the cell. Bacteriophage which do not express the antibody will not bind to the cell. Such panning techniques are well known in the art and are described for example, in Wright et al., (supra).

Processes such as those described above, have been developed for the production of human antibodies using M13 bacteriophage display (Burton et al., 1994, Adv. Immunol. 57:191-280). Essentially, a cDNA library is generated from mRNA obtained from a population of antibody-producing cells. The mRNA encodes rearranged immunoglobulin genes and thus, the cDNA encodes the same. Amplified cDNA is cloned into M13 expression vectors creating a library of phage which express human Fab fragments on their surface. Phage which display the antibody of interest are selected by antigen binding and are propagated in bacteria to produce soluble human Fab immunoglobulin. Thus, in contrast to conventional monoclonal antibody synthesis, this procedure immortalizes DNA encoding human immunoglobulin rather than cells which express human immunoglobulin.

The procedures just presented describe the generation of phage which encode the Fab portion of an antibody molecule. However, the invention should not be construed to be limited solely to the generation of phage encoding Fab antibodies. Rather, phage which encode single chain antibodies (scFv/phage antibody libraries) are also included in the invention. Fab molecules comprise the entire Ig light chain, that is, they comprise both the variable and constant region of the light chain, but include only the variable region and first constant region domain (CHl) of the heavy chain. Single chain antibody molecules comprise a single chain of protein comprising the Ig Fv fragment. An Ig Fv fragment includes only the variable regions of the heavy and light chains of the antibody, having no constant region contained therein. Phage libraries comprising scFv DNA may be generated following the procedures described in Marks et al., 1991, J. Mol. Biol. 222:581-597. Panning of phage so generated for the isolation of a desired antibody is conducted in a manner similar to that described for phage libraries comprising Fab DNA.

The invention should also be construed to include synthetic phage display libraries in which the heavy and light chain variable regions may be synthesized such that they include nearly all possible specificities (Barbas, 1995, Nature Medicine 1:837-839; de Kruif et al., 1995, J. Mol. Biol. 248:97-105).

Polypeptides other than antibodies may be obtained by standard methods known to the skilled artisan. Methods include in vitro peptide synthesis and biological means. Biological means includes purification from a biological source, recombinant synthesis and in vitro translation systems.

Merrifield-type solid phase peptide synthesis may be routinely performed to yield peptides up to about 60-70 residues in length, and may, in some cases, be utilized to make peptides up to about 100 amino acids long. Larger peptides may also be generated synthetically via fragment condensation or native chemical ligation (Dawson et al., Ann. Rev. Biochem. 69:923-60 (2000)). A great advantage to the utilization of a synthetic peptide route is the ability to produce large amounts of peptides, even those that rarely occur naturally, with relatively high purities, i.e., purities sufficient for research, diagnostic or therapeutic purposes.

Examples of solid phase peptide synthesis methods include the BOC method which utilized tert-butyloxcarbonyl as the α-amino protecting group, and the FMOC method which utilizes 9-fluorenylmethyloxcarbonyl to protect the α-amino of the amino acid residues, both which methods are well-known by those of skill in the art.

Incorporation of N- and/or C- blocking groups may also be achieved using protocols conventional to solid phase peptide synthesis methods. For incorporation of C-terminal blocking groups, for example, synthesis of the desired peptide is typically performed using, as solid phase, a supporting resin that has been chemically modified so that cleavage from the resin results in a peptide having the desired C-terminal blocking group. To provide peptides in which the C-terminus bears a primary amino blocking group, for instance, synthesis is performed using a p-methylbenzhydrylamine (MBHA) resin, so that, when peptide synthesis is completed, treatment with hydrofluoric acid releases the desired C-terminally amidated peptide. Similarly, incorporation of an N-methylamine blocking group at the C-terminus is achieved using N-methylaminoethyl-derivatized DVB, resin, which upon HF treatment releases a peptide bearing an N-methylamidated C-terminus. Blockage of the C-terminus by esterification can also be achieved using conventional procedures. This entails use of resin/blocking group combination that permits release of side-chain peptide from the resin, to allow for subsequent reaction with the desired alcohol, to form the ester function. FMOC protecting group, in combination with DVB resin derivatized with methoxyalkoxybenzyl alcohol or equivalent linker, can be used for this purpose, with cleavage from the support being effected by TFA in dicholoromethane. Esterification of the suitably activated carboxyl function, e.g. with DCC, can then proceed by addition of the desired alcohol, followed by de-protection and isolation of the esterified peptide product.

Incorporation of N-terminal blocking groups may be achieved while the synthesized peptide is still attached to the resin, for instance by treatment with a suitable anhydride and nitrile. To incorporate an acetyl blocking group at the N-terminus, for instance, the resin-coupled peptide can be treated with 20% acetic anhydride in acetonitrile. The N-blocked peptide product may then be cleaved from the resin, de-protected and subsequently isolated.

Biological preparation of a cell binding moiety or signal factor involves expression of a gene or coding sequence for the molecule. A naturally-occurring cell binding polypeptide or signal factor may be obtained from cells expressing an endogenous gene. Such cells, optionally, may be genetically modified to overexpress an endogenous gene, using standard molecular biology techniques. Genetic modifications to aid in purification, such as the addition of a secretion peptide signal, or a 6-His tag, may also be made to an endogenous gene.

Biological preparation using an exogenous coding sequence, or a coding sequence for a variant thereof, may also be done to generate the molecule. DNA sequences of signal factors are known in the art. For instance, the nucleotide sequence and amino acid sequence of human EGF are available as GenBank Accession Nos. NM_001963 and NP_001954, respectively, incorporated herein by reference in their entirety. Neutral sequence variants of these sequences, based on the degeneracy of the genetic code, are also useful. Similarly, a DNA sequence encoding any polypeptide variant described elsewhere herein is useful for preparing a moiety or signal factor of the composition. Vectors for expression cassettes and methods for the introduction of exogenous DNA into cells with concomitant expression of the exogenous DNA in the cells are described, for example, in Sambrook et al., supra, 2001; Ausubel et al., supra, 2005. Techniques for introducing vectors into target cells include, but are not limited to, electroporation, photoporation, calcium precipitation, fusion, transfection, lipofection, viral targeting and the like.

Any expression vector compatible with the expression of a polypeptide in a host cell is suitable for use in the instant invention, and can be selected from the group consisting of a plasmid DNA, a viral vector, and a mammalian vector. Vectors may be episomal, or may be provided for integration into the target cell genome via homologous recombination or random integration. Viral vectors useful in the methods of the invention include, but are not limited to, cytomegalovirus vectors, adenovirus vectors and retrovirus vectors, such as MigRI, MMLC, HIV-2 and ALV.

The vector comprising the expression cassette, or a vector that is cointroduced with the expression vector, can comprise a marker gene. Marker genes are useful, for instance, to monitor transfection efficiencies. Marker genes include genes for selectable markers, including, but not limited to, G418, hygromycin, and methotrexate, and genes for detectable markers, including, but not limited to luciferase and GFP.

The coding sequence contained in an expression cassette may, optionally, be fused in-frame to other coding sequences. For instance, the coding sequence of an epitope or other detectable tag may be included. Such tags are useful, for instance, to assist in the rapid purification of the encoded polypeptide or variant thereof. An example of such a tag is a 6-His sequence. The fusion may be at either the N-terminal or the C-terminal of a polypeptide, provided the cell binding activity or signal factor activity is maintained.

In the context of an expression vector, the vector may be readily introduced into a host cell, e.g., mammalian, bacterial, yeast or insect cell by any method in the art. For example, the expression vector can be transferred into a host cell by physical, chemical or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al., supra, 2001 and Ausubel et al., supra, 2005.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, e.g., U.S. Patent Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle in vitro and in vivo is a liposome (i.e., an artificial membrane vesicle). The preparation and use of such systems are well known in the art.

To ensure that the polypeptide obtained from either chemical or biological synthetic techniques is the desired polypeptide, analysis of the polypeptide composition should be conducted. Such amino acid composition analysis may be conducted using high resolution mass spectrometry to determine the molecular weight of the peptide. Alternatively, or additionally, the amino acid content of the peptide may be confirmed by hydrolyzing the peptide in aqueous acid, and separating, identifying and quantifying the components of the mixture using HPLC, or an amino acid analyzer. Protein sequenators, which sequentially degrade the peptide and identify the amino acids in order, may also be used to definitively determine the sequence of the peptide.

Prior to use in the composition of the invention, polypeptides are purified to remove contaminants. Any one of a number of a conventional purification procedures may be used to attain the required level of purity including, for example, reversed-phase highpressure liquid chromatography (HPLC) using an alkylated silica column, such as C₄-,C₈- or C₁₈- silica, or variations thereof. A gradient mobile phase of increasing organic content is generally used to achieve purification, for example, acetonitrile in an aqueous buffer, usually containing a small amount of trifluoroacetic acid. Ion-exchange chromatography may be also used to separate polypeptides based on their charge. Gel filtration chromatography may be used to separate polypeptides based on their size.

Substantially pure protein obtained as described herein may be purified by following known procedures for protein purification, wherein an immunological, enzymatic or other assay is used to monitor purification at each stage in the procedure. Protein purification methods are well known in the art, and are described, for example in Deutscher et al. (ed., 1990, Guide to Protein Purification, Harcourt Brace Jovanovich, San Diego).

Hook, anchor or signal factor polypeptides may be modified using ordinary molecular biological techniques to improve their resistance to proteolytic degradation or to optimize solubility properties or to render them more suitable as a therapeutic agent. Analogs of such polypeptides include those containing residues other than naturally occurring L-amino acids, e.g., D-amino acids or non-naturally occurring synthetic amino acids. The polypeptides useful in the invention may further be conjugated to non-amino acid moieties that are useful in their therapeutic application. In particular, moieties that improve the stability, biological half-life, water solubility, and immunologic characteristics of the peptide are useful. A non-limiting example of such a moiety is polyethylene glycol (PEG).

In addition, covalent attachment of biologically active compounds to water-soluble polymers is one method for alteration and control of biodistribution, pharmacokinetics, and often, toxicity for these compounds (Duncan et al., 1984, Adv. Polym. Sci. 57:53-101). Many water-soluble polymers have been used to achieve these effects, such as poly(sialic acid), dextran, poly(N-(2-hydroxypropyl)methacrylamide) (PEPMA), poly(N-vinylpyrrolidone) (PVP), poly(vinyl alcohol) (PVA), poly(ethylene glycol-co-propylene glycol), poly(N-acryloyl morpholine (PAcM), and poly(ethylene glycol) (PEG) (Powell, 1980, Polyethylene glycol. In R. L. Davidson (Ed.) Handbood of Water Soluble Gums and Resins, McGraw-Hill, New York, NY, chapter 18). PEG possess an ideal set of properties: very low toxicity (Pang, 1993, J. Am. Coll. Toxicol. 12: 429-456) excellent solubility in aqueous solution (Powell, supra, 1980), low immunogenicity and antigenicity (Dreborg et al., 1990, Crit. Rev. Ther. Drug Carrier Syst. 6: 315-365). PEG-conjugated or "PEGylated" protein therapeutics, containing single or multiple chains of polyethylene glycol on the protein, have been described in the scientific literature (Clark et al., 1996, J. Biol. Chem. 271: 21969-21977; Hershfield, 1997, Biochemistry and immunology of poly(ethylene glycol)-modified adenosine deaminase (PEG-ADA). In J. M. Harris and S. Zalipsky (Eds) Poly(ethylene glycol): Chemistry and Biological Applications. American Chemical Society, Washington, D.C., p 145-154; Olson et al.,1997, Preparation and characterization of poly(ethylene glycol)ylated human growth hormone antagonist. In J. M. Harris and S. Zalipsky (Eds) Poly(ethylene glycol): Chemistry and Biological Applications. American Chemical Society, Washington, D.C., p 170-181).

Hook, anchor and signaling factor moieties may be modified to enable binding to a common substrate. In one embodiment, each moiety is biotinylated to enable binding to avidin. In a preferred embodiment, a composition of the invention comprises a biotinylated antibody to ICAM-1 and a biotinylated antibody to CD90 bound to avidin. Optionally, biotinylated EGF is also bound to the avidin.

Other cell binding moieties useful as hook and/or anchor molecules may be readily identified by the skilled artisan using standard techniques in the art to assess binding affinity and specificity. Other moieties include small molecules, aptamers, peptides and peptidomimetics. Test candidates for use as cell binding moieties may be obtained using any of the numerous approaches in combinatorial library methods known in the art, including biological libraries, spatially-addressable parallel solid phase or solution phase libraries, synthetic library methods requiring deconvolution, the "one-bead one-compound" library method, and synthetic library methods using affinity chromatography selection. The biological library approach is limited to peptide libraries, while the other four approaches are applicable to peptide, nonpeptide oligomer, or small molecule libraries of compounds (Lam, 1997, Anticancer Drug Des. 12:145).

Examples of methods for the synthesis of molecular libraries may be found in the art, for example, in: DeWitt et al., 1993, Proc. Natl. Acad. Sci. USA 90:6909-6913; Erb et al., 1994, Proc. Natl. Acad. Sci. USA 91:11422-11426; Zuckermann et al., 1994, J. Med. Chem. 37:2678-2685; Cho et al., 1992, Science 261:1303-1305; Carell et al., 1994, Angew. Chem. Int. Ed. Engl. 33:2059-2061; Carell et al., 1994, Angew. Chem. Int. Ed. Engl. 33:2061-2064; and Gallop et al., 1994, J. Med. Chem. 37:1233-1251.

Libraries of compounds may be presented in solution (e.g., Houghten, 1992, Bio/Techniques 13:412-421), or on beads (Lam, 1991, Nature 354:82-84), chips (Fodor, 1993, Nature 364:555-556), bacteria (U.S. Pat. No. 5,223,409), spores (U.S. Pat. Nos. 5,571,698; 5,403,484; and 5,223,409), plasmids (Cull et al., 1992, Proc. Natl. Acad. Sci. USA 89:1865-1869), or phage (Scott and Smith, 1990, Science 249:386-390; Devlin, 1990, Science 249:404-406; Cwirla et al., 1990, Proc. Natl. Acad. Sci. USA 87:6378-6382; and Felici, 1991, J Mol. Biol. 222:301-310).

The hook, anchor and optional signaling factor moieties may be linked directly together, may be linked indirectly together, or combinations thereof. Linkage may be covalent bonding or non-covalent bonding. For example, in an embodiment, the hook and anchor moieties are linked directly together by covalent bonds and are linked to a signaling factor indirectly by non-covalent bonding to a common substrate. In another embodiment, all three moieties are linked directly via covalent bonds. In yet another embodiment, the components are indirectly linked together by binding the individual components to a common substrate. In one aspect of this embodiment, the linkage is via noncovalent bonds; in another aspect, the linkage is via covalent bonds.

The common substrate is preferably a biocompatible material. In some embodiments, a common substrate that is a biodegradable material is preferred. Non-limiting examples of biocompatible materials useful in the invention include avidin, collagen, biodegradable hydrogels, complex sugars, such as starches and alginates, biocompatible polymers, including dendrimers, such as DNA dendrimers, and the like. Avidin is a preferred material for use as a common substrate. One substrate embodiment comprises a biodegradable molecule to which the anchor, hook and optional cell signals may be bound covalently or non-covalently, using techniques known in the art.

Covalent attachments useful in the composition include, but are not limited to, standard protein cross-linking chemistries, such as glutaraldehyde activation of amine-functionalized surfaces, trialkoxy aldehyde silanes, DMP (dimethyl pimelimidate), and N-hydroxysuccinimide active ester. Non-limiting examples of non-covalent attachments useful in preparing the composition of the invention include hydrophobic interactions and avidin/biotin systems. Avidin/biotin systems are preferred.

Avidin (egg white protein) is a globular protein that has four binding sites for the small molecule, biotin. The binding constant of biotin for avidin is very high, ensuring that the binding between any biotinylated molecules and avidin is durable while avidin is intact. Avidin is a naturally-occurring protein, and while eating excessive unbound egg white has been shown to result in biotin deficiency in the absence of biotin, no other side effects have been seen. Although used extensively in in vitro binding assays, there has been limited in vivo use of avidin. Avidin has been shown to degrade in vivo over one to five days, thereby providing ample time for the RCs to be bound to the complex in the proximity of the injury. The limited in vivo duration of avidin is envisioned to be beneficial for compositions administered to treat reperfusion injury by further inhibiting leukocyte activation and reperfusion injury, as well as reducing fibrosis, enhancing neuroprotection and neural tissue repair.

Biotinylation is the process of attaching biotin, or a biotin derivative, to another molecule, for instance an antibody, yielding a biotinylated molecule. Biotinylation as used herein encompasses both chemical conjugation of biotin to a molecule, directly or via a linker molecule, recombinant biotinylation, as well as indirect biotinylation. A moiety may be indirectly labeled by binding it with a biotinylated reagent. For example, the biotinylated reagent may be a biotinylated antibody that specifically binds to the moiety. This method is suitable in the instant invention, provided the bound antibody does not interfere with the moiety's ability to bind its target molecule or to carry out its signaling function.

Biotinylated linkers are well known in the art and are commercially available. The biotin can be separated by any length linker from the moiety attachment site. Linkers are advantageous in reducing potential interactions between the biotin and the molecule to which it is conjugated, and also enhances biotin binding to the biotin binding sites of avidin, which are relatively deep.

Compounds useful in conjugating a molecule with biotin include, but are not limited to, aliphatic amines, carboxylic acid, DNP-X-biocytin-X, FMOC, hydrazide, iodoacetamide, maleimide, nitriloacetic acid and succinimidyl ester. Biotin, including various spacers, linking groups and the like, and methods of biotinylation are well known to the skilled artisan. See, for example, Savage et al., 1992, Avidin-Biotin Chemistry: A Handbook, Pierce Chemical Company, Rockford, IL; Diamandis et al., 1991, Clin. Chem. 37:625-636; DE 3629194; U.S. Pat. Nos. 4,709,037, 4,794,082, 4,798,795, 5,180,828, and 5,252,743; and WO 85/05638, each of which is incorporated herein by reference in its entirety.

In vivo biotinylation can be accomplished by recombinant methods known in the art. In brief, a nucleic acid encoding a polypeptide to be biotinylated is operably linked to a sequence encoding a biotinylation signal, such as Avitag (Beckett et al., 1999, Prot. Sci. 8:921-929) or Biotab (de Boer et al., 2003, PNAS 100:7480-7485). The recombinant nucleic acid is then expressed in a host cell which expresses a biotin ligase (e.g. E. coli Bir A), either endogenously or recombinantly and is cultured in a biotin-containing medium. U.S. Pat. Publication No. 20040033603, hereby incorporated by reference in its entirety, discloses bicistronic vectors useful in such in vivo biotinylation applications. Alternatively, the recombinant nucleic acid encoding a polypeptide sequence fused to a biotinylation signal is expressed in a host cell in the absence of biotin. The fusion protein is purified and is biotinylated in vitro using isolated biotin ligase. Products for in vivo biotinylation systems are commercially available, for instance, from GeneCopeia and Avidity.

In preparing compositions of the invention, each unit of the repair composition comprises at least one hook and at least one anchor (1:1). However, other ratios of hook and anchor molecules are envisioned. For instance, the ratio could be 2 anchors to 1 hook. In this composition, the 2 anchors may bind the identical molecule or may bind different molecules. Furthermore, the ratio of hook, anchor and optional signaling factor may be 1:1:1, however, the composition is not limited to this ratio.

When the moieties of the composition are polypeptides, peptide coupling chemistry may be employed to link the moieties together directly or indirectly by means of a linking agent. The standard peptide coupling chemistry methods and procedures useful in this invention are readily available. Examples of books using these methods include, but are not limited to, the following citations incorporated herein by reference: P. D. Bailey, An Introduction to Peptide Chemistry, Ed.: John Wiley & Sons, 1990; Miklos Bodansky, Peptide Chemistry, A Practical Textbook, Ed.: Springer-Verlag, 1988; Miklos Bodansky, Principles of Peptide Synthesis, "Reactivity and Structure Concepts in Organic Chemistry," Volume 16, Ed.: Springer-Verlag, 1984; and Miklos Bodansky, Principles of Peptide Synthesis, "Reactivity and Structure Concepts in Organic Chemistry," Volume 21, Ed.: Springer-Verlag, 1984. See also U.S. Pat. Nos. 4,340,535 and 5,776,427 and EP 44167, each of which is incorporated herein by reference in its entirety.

Cross-linking reagents are used to form molecular bridges that tie together functional groups of two different proteins (e.g., a hook moiety and a anchor moiety). To link two different proteins in a step-wise manner, heterobifunctional cross-linkers can be used which eliminate the unwanted homopolymer formation. An exemplary heterobifunctional cross-linker contains two reactive groups: one reacting with primary amine group (e.g., N-hydroxy succinimide) and the other reacting with a thiol group (e.g., pyridyl disulfide, maleimides, halogens, etc.). Through the primary amine reactive group, the cross-linker can react with the lysine residue(s) of one protein (e.g., the selected antibody or fragment) and through the thiol reactive group, the cross-linker, already tied up to the first protein, reacts with the cysteine residue (free sulfhydryl group) of the other protein. Useful heterobifunctional crosslinking agents include 4-succinimidyloxycarbonyl-methyl-(2-pyridyldithio)-toluene (SMPT) or N-succinimidyl-3-(2-pyridyidithio)propionate (SPDP), both of which can be obtained from Pierce, Rockland, Ill.

SMPT is a bifunctional cross-linker containing a disulfide bond that is "sterically hindered" by an adjacent benzene ring and methyl groups. It is believed that steric hindrance of the disulfide bond serves a function of protecting the bond from attack by thiolate anions, such as glutathione, which can be present in tissues and blood, and thereby help in preventing decoupling of linked moieties. The SMPT cross-linking reagent, as with many other known cross-linking reagents, lends the ability to cross-link functional groups such as the SH of cysteine or primary amines (e.g., the epsilon amino group of lysine). Another possible type of cross-linker includes the heterobifunctional photoreactive phenylazides containing a cleavable disulfide bond such as sulfosuccinimidyl-2-(p-azido salicylamido) ethyl-1,3'-dithiopropionate. The N-hydroxy-succinimidyl group reacts with primary amino groups and the phenylazide (upon photolysis) reacts non-selectively with any amino acid residue. Crosslinking reagents that include a means to bind to a common substrate are also useful. For instance, 2-[N^{α}-benzoylbenzoicamido-N⁶-(6-biotinamidocaproyl)-L-lysinylamido]ethyl methanethiosulfonate (MTS-BP-Bio; Toronto Research Chemicals) is a biotinylated bifunctional crosslinker. Two moieties may be crosslinked to it and this conjugate may then be non-covalently bound to avidin.

While numerous types of disulfide-bond containing linkers are known that can successfully be employed to conjugate a moiety to a substrate, certain linkers may generally be preferred over other linkers, based on differing pharmacologic characteristics and capabilities. For example, linkers that contain a disulfide bond that is sterically "hindered" may be preferred, due to their greater stability in vivo, thus preventing release of the active agent prior to binding at the site of action. However, non-hindered linkages, such as SATA and 2-iminothiolane, may also be used. Other crosslinkers, including trifunctional crosslinkers, such as tris-succinimidyl aminotriacetate (TSAT), may be used in preparing the composition of the invention.

The spacer arm between the reactive groups of any cross-linkers can have various length and chemical composition. A longer spacer arm allows a better flexibility of the composition components while some particular features in the bridge (e.g., benzene group) can lend extra stability to the reactive group or an increased resistance of the chemical link to the action of various aspects (e.g., disulfide bond resistant to reducing agents).

In addition to chemical conjugation of two components, polypeptide moieties may also be directly linked together as a fusion protein, provided the moieties retain their binding capacity in the context of the fusion protein. The binding moieties may be separated within the fusion protein by a spacer peptide to enable proper folding of the moieties and to reduce potential steric problems when the fusion protein is bound to both binding targets. Using standard molecular biology techniques, a nucleic acid encoding a polypeptide comprising, for instance, a hook moiety and an anchor moiety, may be used to produce a fusion protein. The nucleic acid molecules are inserted into a vector that is able to express the encoded fusion protein when introduced into an appropriate host cell. The nucleic acid molecules are operably linked to promoter/regulatory sequences. Appropriate host cells include, but are not limited to, bacterial, yeast, insect, and mammalian cells. Any of the methods known to one skilled in the art for the insertion of DNA fragments into a vector may be used to construct expression vectors encoding the fusion proteins of the invention. under control of transcriptional/translational control signals. These methods may include in vitro recombinant DNA and synthetic techniques and in vivo recombinations. Promoters which may be used to control expression of the fusion polypeptide molecules include, but are not limited to, the long terminal repeat (Squinto et al., 1991, Cell 65:1 20_; the SV40 early promoter region, the CMV promoter, the M-MuLV 5' terminal repeat the promoter contained in the 3' long terminal repeat of Rous sarcoma virus, the herpes thymidine kinase promoter, the regulatory sequences of the metallothionine gene; prokaryotic expression vectors such as the β-lactamase promoter, or the tac promoter; promoter elements from yeast or fungi such as the Gal 4 promoter, the ADH (alcohol dehydrogenase) promoter, PGK (phosphoglycerol kinase) promoter, alkaline phosphatase promoter, and tissue-specific transcriptional control regions obtained from, for example, an elastase I gene, insulin gene, immunoglobulin gene, mouse mammary tumor virus, albumin gene, α-fetoprotein gene, α1-antitrypsin gene, β-globin gene, myelin basic protein gene, myosin light chain-2 gene, and gonadotropic releasing hormone gene.

In preparing the composition of the invention, a fusion protein may be modified as described elsewhere herein to enable binding to a common substrate. A fusion protein may also be linked to another moiety, for example, an optional signaling factor, by means of cross-linking as previously described.

The composition of the invention may be purifed to remove contaminants, such as unbound or unconjugated moieties or substrate, using standard purification techniques known in the art. Such techniques may include, but are not limited to, gel filtration, high performance liquid chromatography, molecular exclusion chromatograpy and affinity chromatograghy.

In some embodiments, the composition is bound to a matrix. These embodiments are particularly useful for treating structural tissue injuries or any other injuries in which liquid formulations of the repair composition are not advantageous. Matrices suitable for use with the composition of the invention include resorbable mesh of polyglactin 910 (Vicryl^{®}, Ethicon, Somerville, NJ), dextran, polyglycolic acid (PGLA), decellularized small intestinal lining (SIS^{®}, DePuy, Warsaw, IN), woven hyaluronic acid mesh (HYAFF^{®}, Fidia Advanced Biopolymers, Abano Terme, Italy), and collagen matrix (Gelfoam^{®}, Pfizer, New York, NY). The anchor and hook moieties and the optional signaling factor may be covalently or non-covalently attached to a matrix material. Alternatively, the anchor and hook moieties and the optional signaling factor may be covalently or non-covalently attached to a carrier (such as dextran or avidin), which is then covalently bound to a resorbable matrix mesh or non-covalently adsorbed onto a resorbable matrix mesh.

In some embodiments, useful for treating skin injuries, suitable matrices include a resorbable Vicryl^{®} matrix mesh, PGLA, decellularized small intestinal lining (SIS^{®}), woven hyaluronic acid mesh (HYAFF^{®}), or collagen matrix (Gelfbam^{®}) with the anchor and hook moieties covalently or non-covalently attached. Alternative matrix materials include acellular skin grafting materials (Transcyte^{®}, Smith&Nephew, Hull, UK) or living skin grafting materials, such as Dertnagraft^{®} (Smith&Nephew) or Apligraf^{®} (Organogenesis, Canton, MA). Alternatively, a carrier, such as dextran or avidin, with covalently or non-covalently bound anchor and hook moieties is seeded onto the matrix.

### Methods of use

The compositions of the invention are beneficially used for the treatment of injured cells or tissues in numerous different medical conditions. A non-limiting list of injuries include structural tissue injury, such as cartilage and meniscal damage, ischemic injury, including that connected to stroke, myocardial infarction, heart attack, spinal cord injury, donor organ injury, organ transplant recipient, reperfusion, vascular stenting, transient ischemic attack, chronic and acute mesenteric ischemia, critical limb ischemia, cancer, bone marrow injuries and renal damage.

Such methods may be carried out in any animal in need of treatment. Preferably, the animal is a mammal, more preferably a primate and more preferably still, a human. Thus, although the descriptions of pharmaceutical compositions provided herein are principally directed to pharmaceutical compositions which are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include, but are not limited to, humans and other primates, mammals including commercially relevant mammals such as cattle, pigs, horses, sheep, cats, and dogs, birds including commercially relevant birds such as chickens, ducks, geese, and turkeys.

In the repair of injuries of structural tissues, such as cartilage, bone, soft tissues and skin, injuries including articular cartilage or meniscal injuries may be treated using a composition of the invention. The hook, anchor and optional signaling factor moieties used in the composition of the invention may be selected from those in Table 1, but are not limited to those in Table 1. In an embodiment, the composition comprises a hook, an anchor and an optional cell signal molecule bound to a pharmaceutically-acceptable structural substrate. The composition may be applied to the site of injury by way of an open surgical procedure or endoscopic procedure.

In yet another embodiment, compositions of the present invention may be administered to a subject before, during or after injury an ischemic injury. Ischemic injury is generally caused by an occlusive event in the blood vessel supplying the tissue. When the vessel opens up, or is reopened through therapeutic intervention, often further damage is created in the tissue by the influx of not only scar-forming cells, but also inflammatory cells which release destructive enzymes (reperfusion injury). In previous therapies for reperfusion injury, treatments have primarily targeted the inhibition of leukocyte localization and binding to the vascular tissues. Early studies with infusion of growth factors (VEGF and EPO) have demonstrated limited success in patients, presumably due to the small number of endogenous number of cells at the site.

In another embodiment, compositions of the present invention may be administered to a subject who has, or is subject to, due to medical disorders, medical treatments or toxin exposure, renal damage. Renal damage encompasses renal failure, both acute and chronic, acute tubular necrosis (ATN), renal artery stenosis, ischemic nephropathy, vasculitis, focal segmental glomerulosclerosis, IgA nephritis, lupus nephritis, polycystic kidney disease, chronic tubulointerstitial nephritis and reflux nephropathy. The most common causes of chronic renal failure are diabetic nephropathy, hypertension and glomerulonephritis. Compositions may be administered intravenously or locally, for instance, via a ureter catheter. Compositions may be administered by dialysis. The composition may be administered once, multiple times, or in an on-going manner, for instance for chronic disorders. The skilled artisan can readily determine the dosing and administration without undue experimentation.

Compositions may be administered to a stroke, heart attack or spinal cord injury victim in order to reduce the severity of cell and tissue loss during the post-ischemic event period. Compositions of the present invention may be administered before, during and/or after any medical procedures that carry risk of reperfusion injury during revascularization procedures, such as placement of a stent after myocardial ischemia, coronary bypass grafting, and organ transplantation of heart, liver, lung, pancreas and kidney. In each of these cases, the composition is preferably administered intravenously either distant or local to the site of revascularization.

Repeated bouts of ischemia and reperfusion injury are thought to be a factor leading to the formation and failure to heal of chronic wounds, such as pressure sores and diabetic foot ulcers. Accordingly, chronic wounds may be treated by administering a composition of the invention.

A patient undergoing intestinal surgery may be treated with the inventive composition prior to, contemporaneously with and/or after blood flow is restored to the intestine, in order to preclude or reduce reperfusion injury.

The composition of the invention also is useful in conditioning organs and tissues undergoing preparation for transplant. In one embodiment, a donor heart may be contacted with the inventive tissue repair compositions before, during, and/or after implantation such that injury, including, without limitation, reperfusion injury, is reduced in the period after transplant. The recipient of the transplant may also be treated with the tissue repair composition, before, during and/or after the transplantation.

The composition of the invention is useful for cancer immunotherapy treatments. In one embodiment, a patient diagnosed with a cancer is administered a repair composition comprising a moiety the binds to a tumor specific antigen for the cancer and a moiety that binds to an immune cell, preferably to a helper T cell, a killer T cell or natural killer cell. The composition optionally further comprises an cytokine. The composition may also comprise a moiety the binds co-stimulatory molecules, such as CDw137, B7-H2 and others described elsewhere herein and known in the art.

The composition may be administered systemically or locally. The administration may be by intravenous infusion; intra-arterial infusion; intraperitoneal injection; direct local injection to a tissue; subcutaneous injection; or intramuscular injection. Pumps may be used for continuous delivery. Various delivery devices are available, including intravenous infusion sets, syringes, infusion catheters, intra-vessel balloon-based delivery catheters and stents. The composition of the present invention may also be delivered via a matrix or scaffold. Biodegradable polymers may be used as a matrix. Hydrogels may be employed with varying degrees of crosslinking.

The dosage of the repair compostion varies within wide limits and may be adjusted to the individual requirements in each particular case. The dosage depends on the condition treated, the weight and general state of health of the recipient, the number and frequency of administrations and other variables known to those of skill in the art. For most indications, the dose of the repair composition in soluble form will vary between 5 µg and 1 g per kg per day.

The invention encompasses the preparation and use of pharmaceutical compositions comprising a composition of the invention useful for treatment of the diseases disclosed herein as an active ingredient. Such a pharmaceutical composition may consist of the active ingredient alone, in a form suitable for administration to a subject, or the pharmaceutical composition may comprise the active ingredient and one or more pharmaceutically acceptable carriers, one or more additional ingredients, or some combination of these.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

Pharmaceutical compositions that are useful in the methods of the invention may be prepared, packaged, or sold in formulations suitable for oral, rectal, vaginal, parenteral, topical, pulmonary, intranasal, buccal, ophthalmic, intrathecal or another route of administration. Other contemplated formulations include projected nanoparticles, liposomal preparations, resealed erythrocytes containing the active ingredient, and immunologicallybased formulations.

A pharmaceutical composition of the invention may be prepared, packaged, or sold in bulk, as a single unit dose, or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, one-half or one-third of such a dosage.

The relative amounts of the active ingredient, the pharmaceutically acceptable carrier, and any additional ingredients in a pharmaceutical composition of the invention will vary, depending upon the identity, size, and condition of the subject treated and further depending upon the route by which the composition is to be administered. By way of example, the composition may comprise between 0.1% and 100% (w/w) active ingredient.

In addition to the active ingredient, a pharmaceutical composition of the invention may further comprise one or more additional pharmaceutically active agents.

Controlled- or sustained-release formulations of a pharmaceutical composition of the invention may be made using conventional technology.

A formulation of a pharmaceutical composition of the invention suitable for oral administration may be prepared, packaged, or sold in the form of a discrete solid dose unit including, but not limited to, a tablet, a hard or soft capsule, a cachet, a troche, or a lozenge, each containing a predetermined amount of the active ingredient. Other formulations suitable for oral administration include, but are not limited to, a powdered or granular formulation, an aqueous or oily suspension, an aqueous or oily solution, or an emulsion.

As used herein, an "oily" liquid is one which comprises a carbon-containing molecule and which exhibits a less polar character than water.

A tablet comprising the active ingredient may, for example, be made by compressing or molding the active ingredient, optionally with one or more additional ingredients. Compressed tablets may be prepared by compressing, in a suitable device, the active ingredient in a free-flowing form such as a powder or granular preparation, optionally mixed with one or more of a binder, a lubricant, an excipient, a surface active agent, and a dispersing agent Molded tablets may be made by molding, in a suitable device, a mixture of the active ingredient, a pharmaceutically acceptable carrier, and at least sufficient liquid to moisten the mixture. Pharmaceutically acceptable excipients used in the manufacture of tablets include, but are not limited to, inert diluents, granulating and disintegrating agents, binding agents, and lubricating agents. Known dispersing agents include, but are not limited to, potato starch and sodium starch glycolate. Known surface active agents include, but are not limited to, sodium lauryl sulphate. Known diluents include, but are not limited to, calcium carbonate, sodium carbonate, lactose, microcrystalline cellulose, calcium phosphate, calcium hydrogen phosphate, and sodium phosphate. Known granulating and disintegrating agents include, but are not limited to, corn starch and alginic acid. Known binding agents include, but are not limited to, gelatin, acacia, pre-gelatinized maize starch, polyvinylpyrrolidone, and hydroxypropyl methylcellulose. Known lubricating agents include, but are not limited to, magnesium stearate, stearic acid, silica, and talc.

Tablets may be non-coated or they may be coated using known methods to achieve delayed disintegration in the gastrointestinal tract of a subject, thereby providing sustained release and absorption of the active ingredient. By way of example, a material such as glyceryl monostearate or glyceryl distearate may be used to coat tablets. Further by way of example, tablets may be coated using methods described in U.S. Pat. Nos. 4,256,108; 4,160,452; and 4,265,874 to form osmotically-controlled release tablets. Tablets may further comprise a sweetening agent, a flavoring agent, a coloring agent, a preservative, or some combination of these in order to provide pharmaceutically elegant and palatable preparation.

Hard capsules comprising the active ingredient may be made using a physiologically degradable composition, such as gelatin. Such hard capsules comprise the active ingredient, and may further comprise additional ingredients including, for example, an inert solid diluent such as calcium carbonate, calcium phosphate, or kaolin.

Soft gelatin capsules comprising the active ingredient may be made using a physiologically degradable composition, such as gelatin. Such soft capsules comprise the active ingredient, which may be mixed with water or an oil medium such as peanut oil, liquid paraffin, or olive oil.

Liquid formulations of a pharmaceutical composition of the invention which are suitable for oral administration may be prepared, packaged, and sold either in liquid form or in the form of a dry product intended for reconstitution with water or another suitable vehicle prior to use.

Liquid suspensions may be prepared using conventional methods to achieve suspension of the active ingredient in an aqueous or oily vehicle. Aqueous vehicles include, for example, water and isotonic saline. Oily vehicles include, for example, almond oil, oily esters, ethyl alcohol, vegetable oils such as arachis, olive, sesame, or coconut oil, fractionated vegetable oils, and mineral oils such as liquid paraffin. Liquid suspensions may further comprise one or more additional ingredients including, but not limited to, suspending agents, dispersing or wetting agents, emulsifying agents, demulcents, preservatives, buffers, salts, flavorings, coloring agents, and sweetening agents. Oily suspensions may further comprise a thickening agent. Known suspending agents include, but are not limited to, sorbitol syrup, hydrogenated edible fats, sodium alginate, polyvinylpyrrolidone, gum tragacanth, gum acacia, and cellulose derivatives such as sodium carboxymethylcellulose, methylcellulose, hydroxypropylmethylcellulose. Known dispersing or wetting agents include, but are not limited to, naturally-occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (e.g. polyoxyethylene stearate, heptadecaethyleneoxycetanol, polyoxyethylene sorbitol monooleate, and polyoxyethylene sorbitan monooleate, respectively). Known emulsifying agents include, but are not limited to, lecithin and acacia. Known preservatives include, but are not limited to, methyl, ethyl, or n-propyl-para- hydroxybenzoates, ascorbic acid, and sorbic acid. Known sweetening agents include, for example, glycerol, propylene glycol, sorbitol, sucrose, and saccharin. Known thickening agents for oily suspensions include, for example, beeswax, hard paraffin, and cetyl alcohol.

Liquid solutions of the active ingredient in aqueous or oily solvents may be prepared in substantially the same manner as liquid suspensions, the primary difference being that the active ingredient is dissolved, rather than suspended in the solvent. Liquid solutions of the pharmaceutical composition of the invention may comprise each of the components described with regard to liquid suspensions, it being understood that suspending agents will not necessarily aid dissolution of the active ingredient in the solvent. Aqueous solvents include, for example, water and isotonic saline. Oily solvents include, for example, almond oil, oily esters, ethyl alcohol, vegetable oils such as arachis, olive, sesame, or coconut oil, fractionated vegetable oils, and mineral oils such as liquid paraffin.

Powdered and granular formulations of a pharmaceutical preparation of the invention may be prepared using known methods. Such formulations may be administered directly to a subject, used, for example, to form tablets, to fill capsules, or to prepare an aqueous or oily suspension or solution by addition of an aqueous or oily vehicle thereto. Each of these formulations may further comprise one or more of dispersing or wetting agent, a suspending agent, and a preservative. Additional excipients, such as fillers and sweetening, flavoring, or coloring agents, may also be included in these formulations.

A pharmaceutical composition of the invention may also be prepared, packaged, or sold in the form of oil-in-water emulsion or a water-in-oil emulsion. The oily phase may be a vegetable oil such as olive or arachis oil, a mineral oil such as liquid paraffin, or a combination of these. Such compositions may further comprise one or more emulsifying agents such as naturally occurring gums such as gum acacia or gum tragacanth, naturally-occurring phosphatides such as soybean or lecithin phosphatide, esters or partial esters derived from combinations of fatty acids and hexitol anhydrides such as sorbitan monooleate, and condensation products of such partial esters with ethylene oxide such as polyoxyethylene sorbitan monooleate. These emulsions may also contain additional ingredients including, for example, sweetening or flavoring agents.

A pharmaceutical composition of the invention may be prepared, packaged, or sold in a formulation suitable for rectal administration. Such a composition may be in the form of, for example, a suppository, a retention enema preparation, and a solution for rectal or colonic irrigation.

Suppository formulations may be made by combining the active ingredient with a non-irritating pharmaceutically acceptable excipient which is solid at ordinary room temperature (i.e. about 20°C) and which is liquid at the rectal temperature of the subject (i.e. about 37°C in a healthy human). Suitable pharmaceutically acceptable excipients include, but are not limited to, cocoa butter, polyethylene glycols, and various glycerides. Suppository formulations may further comprise various additional ingredients including, but not limited to, antioxidants and preservatives.

Retention enema preparations or solutions for rectal or colonic irrigation may be made by combining the active ingredient with a pharmaceutically acceptable liquid carrier. As is well known in the art, enema preparations may be administered using, and may be packaged within, a delivery device adapted to the rectal anatomy of the subject. Enema preparations may further comprise various additional ingredients including, but not limited to, antioxidants and preservatives.

A pharmaceutical composition of the invention may be prepared, packaged, or sold in a formulation suitable for vaginal administration. Such a composition may be in the form of, for example, a suppository, an impregnated or coated vaginally-insertable material such as a tampon, a douche preparation, or gel or cream or a solution for vaginal irrigation.

Methods for impregnating or coating a material with a chemical composition are known in the art, and include, but are not limited to methods of depositing or binding a chemical composition onto a surface, methods of incorporating a chemical composition into the structure of a material during the synthesis of the material (i.e. such as with a physiologically degradable material), and methods of absorbing an aqueous or oily solution or suspension into an absorbent material, with or without subsequent drying.

Douche preparations or solutions for vaginal irrigation may be made by combining the active ingredient with a pharmaceutically acceptable liquid carrier. As is well known in the art, douche preparations may be administered using, and may be packaged within, a delivery device adapted to the vaginal anatomy of the subject. Douche preparations may further comprise various additional ingredients including, but not limited to, antioxidants, antibiotics, antifungal agents, and preservatives.

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intraperitoneal, intramuscular, intrasternal injection, and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multi-dose containers containing a preservative. Formulations for parenteral administration include, but are not limited to, suspensions, solutions, emulsions in oily or aqueous vehicles, pastes, and implantable sustained-release or biodegradable formulations. Such formulations may further comprise one or more additional ingredients including, but not limited to, suspending, stabilizing, or dispersing agents. In one embodiment of a formulation for parenteral administration, the active ingredient is provided in dry (i.e. powder or granular) form for reconstitution with a suitable vehicle (e.g. sterile pyrogen-free water) prior to parenteral administration of the reconstituted composition.

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono- or di-glycerides. Other parentally-administrable formulations which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt.

Formulations suitable for topical administration include, but are not limited to, liquid or semi-liquid preparations such as liniments, lotions, oil-in-water or water-in-oil emulsions such as creams, ointments or pastes, and solutions or suspensions. Topically-administrable formulations may, for example, comprise from about 1% to about 10% (w/w) active ingredient, although the concentration of the active ingredient may be as high as the solubility limit of the active ingredient in the solvent. Formulations for topical administration may further comprise one or more of the additional ingredients described herein.

A pharmaceutical composition of the invention may be prepared, packaged, or sold in a formulation suitable for pulmonary administration via the buccal cavity. Such a formulation may comprise dry particles which comprise the active ingredient and which have a diameter in the range from about 0.5 to about 7 nanometers, and preferably from about to about 6 nanometers. Such compositions are conveniently in the form of dry powders for administration using a device comprising a dry powder reservoir to which a stream of propellant may be directed to disperse the powder or using a self-propelling solvent/powder-dispensing container such as a device comprising the active ingredient dissolved or suspended in a low-boiling propellant in a sealed container. Preferably, such powders comprise particles wherein at least 98% of the particles by weight have a diameter greater than 0.5 nanometers and at least 95% of the particles by number have a diameter less than 7 nanometers. More preferably, at least 95% of the particles by weight have a diameter greater than 1 nanometer and at least 90% of the particles by number have a diameter less than 6 nanometers. Dry powder compositions preferably include a solid fine powder diluent such as sugar and are conveniently provided in a unit dose form.

Low boiling propellants generally include liquid propellants having a boiling point of below 65°F at atmospheric pressure. Generally the propellant may constitute 50 to 99.9% (w/w) of the composition, and the active ingredient may constitute 0.1 to 20% (w/w) of the composition. The propellant may further comprise additional ingredients such as a liquid non-ionic or solid anionic surfactant or a solid diluent (preferably having a particle size of the same order as particles comprising the active ingredient).

Pharmaceutical compositions of the invention formulated for pulmonary delivery may also provide the active ingredient in the form of droplets of a solution or suspension. Such formulations may be prepared, packaged, or sold as aqueous or dilute alcoholic solutions or suspensions, optionally sterile, comprising the active ingredient, and may conveniently be administered using any nebulization or atomization device. Such formulations may further comprise one or more additional ingredients including, but not limited to, a flavoring agent such as saccharin sodium, a volatile oil, a buffering agent, a surface active agent, or a preservative such as methylhydroxybenzoate. The droplets provided by this route of administration preferably have an average diameter in the range from about 0.1 to about 200 nanometers.

The formulations described herein as being useful for pulmonary delivery are also useful for intranasal delivery of a pharmaceutical composition of the invention.

Another formulation suitable for intranasal administration is a coarse powder comprising the active ingredient and having an average particle from about 0.2 to 500 micrometers. Such a formulation is administered in the manner in which snuff is taken i.e. by rapid inhalation through the nasal passage from a container of the powder held close to the nares.

Formulations suitable for nasal administration may, for example, comprise from about as little as 0.1% (w/w) and as much as 100% (w/w) of the active ingredient, and may further comprise one or more of the additional ingredients described herein.

A pharmaceutical composition of the invention may be prepared, packaged, or sold in a formulation suitable for buccal administration. Such formulations may, for example, be in the form of tablets or lozenges made using conventional methods, and may, for example, 0.1 to 20% (w/w) active ingredient, the balance comprising an orally dissolvable or degradable composition and, optionally, one or more of the additional ingredients described herein. Alternately, formulations suitable for buccal administration may comprise a powder or an aerosolized or atomized solution or suspension comprising the active ingredient. Such powdered, aerosolized, or aerosolized formulations, when dispersed, preferably have an average particle or droplet size in the range from about 0.1 to about 200 nanometers, and may further comprise one or more of the additional ingredients described herein.

A pharmaceutical composition of the invention may be prepared, packaged, or sold in a formulation suitable for ophthalmic administration. Such formulations may, for example, be in the form of eye drops including, for example, a 0.1-1.0% (w/w) solution or suspension of the active ingredient in an aqueous or oily liquid carrier. Such drops may further comprise buffering agents, salts, or one or more other of the additional ingredients described herein. Other opthalmically-administrable formulations which are useful include those which comprise the active ingredient in microcrystalline form or in a liposomal preparation.

As used herein, "additional ingredients" include, but are not limited to, one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials. Other additional ingredients which may be included in the pharmaceutical compositions of the invention are known in the art and described, for example in Genaro, ed., 1985, Remington's Pharmaceutical Sciences, Mack Publishing Co., Easton, PA, which is incorporated herein by reference.

Typically dosages of the composition of the invention in soluble form which may be administered to an animal, preferably a human, range in amount from about 5 µg to about 1 g per kilogram of body weight of the animal. While the precise dosage administered will vary depending upon any number of factors, including but not limited to, the type of animal and type of disease state being treated, the age of the animal and the route of administration. Preferably, the dosage of the composition will vary from about 20 µg to about 250 mg per kilogram of body weight of the animal and more preferably, from about 30 µg to about 25 mg per kilogram of body weight. For administration of the composition of the invention in a matrix-bound formulation, for example bound to Vicryl®, the concentration of repair composition in the matrix will range in amount from about 1 µg per cm³ of the matrix to about 1 gram per cm³ of the matric.

The composition may be administered to an animal as frequently as several times daily, or it may be administered less frequently, such as once, once a day, once a week, once every two weeks, once a month, or even less frequently, such as once every several months or even once a year or less. The frequency of the dose will be readily apparent to the skilled artisan and will depend upon any number of factors, such as, but not limited to, the type and severity of the disease being treated, the type and age of the animal, etc.

### EXPERIMENTAL EXAMPLES

The invention is now described with reference to the following examples. These examples are provided for the purpose of illustration only and the invention should in no way be construed as being limited to these examples but rather should be construed to encompass any and all variations which become evident as a result of the teaching provided herein.

### Experimental Example 1

The materials and methods used in the experiments presented in this Example are now described.

Animal use was performed under a protocol approved by the IACUC, Molecular Medicine Research Institute, Sunnyvale, CA.

The repair composition (repair composition 1) was created by mixing FITC-conjugated natural avidin (VWR) with biotinylated EGF (Invitrogen); biotinylated anti-rat ICAM-1 (CD54) and biotinylated anti-rat CD90 (VWR) in a stoichiometric ratio of 1M anti-ICAM-1: 1M anti-CD90: 2MEGF: 1M FITC-conjugated natural avidin. This resulted in a final concentration of FITC-conjugated natural avidin of 22 µg/ml, final concentration of the respective antibodies of 43 µg/ml and final concentration of EGF of 4.3 µg/ml in the mixture. The mixture was allowed to incubate at room temperature for 20 minutes and then was diluted 3-fold with PBS.

Rat bone marrow was aspirated from the femurs of 2 male Sprague-Dawley rats, obtained from Charles River Laboratories. The cells were washed once in Dulbecco's-Phosphate buffered saline (D-PBS) and then layered onto a Ficoll-Hypaque density gradient and centrifuged at 400g for 20 minutes. The mononuclear cells at the interface were collected and washed twice with D-PBS.

The results of this experimental example are now described.

One (1) million cells were combined with 100 µl of the repair composition solution for 30 minutes at room temperature, and then washed with PBS at 200g. The cells were examined under fluorescent light microscope to assess binding of the FITC-labeled composition to the cells. Under fluorescent light microscopy, cells with evidence of FITC-labeled composition bound to them was detected. The frequency of such cells was about 5%, which is consistent with the expected frequency of CD90⁺ bone marrow cells in the population of mononuclear cells. This result demonstrates that although bound to avidin, the anti-CD90 antibody was still functional in binding to the CD90 surface antigen.

Due to the high level of cross-reactivity between human and rat ICAM-1, the anti-rat ICAM-1 antibody was expected to bind to human ICAM-1. To verify the binding ability of the avidin-bound anti-ICAM-1 antibody, 100 µl of the repair composition solution was incubated with 300,000 PC3 cells (human prostate cancer cells expressing ICAM-1 (CD54); gift from Dr. Lewis of MMRI, Sunnyvale, CA). Under fluorescent light microscopy, FITC-labeled composition bound to the PC3 cells was detected, evidence that the anti-ICAM antibody is capable of specific binding in the presence of avidin.

### Experimental Example 2

The materials and methods used in the experiments presented in this Example are now described.

Repair composition 1 was prepared as described in Experimental Example 1, however avidin that was not FITC labeled was used. The mixture of avidin, anti-rat ICAM-1, anti-CD90 and EGF was allowed to incubate at room temperature for at least 20 minutes to form the composition, and then the solution was diluted 3-fold with PBS (resulting in a concentration of about 23 µg of components per milliliter). 100 µl of repair composition solution was loaded into 27 gauge syringes for administration.

Adult male Sprague-Dawley rats were obtained from Charles River Laboratories. Animals weighed 300 to 400 gm and were approximately 24-30 weeks of age when used in the current experiments.

Transient (1 hour) middle cerebral artery occlusion (tMCAO) which represents a stroke deficit of intermediate severity with reperfusion injury, was induced using a previously described method of external and intraluminal vascular occlusion (Chen et al., 1992, J Cereb Blood Flow Metab 12(4): 621-8). In brief, rats were anesthetized and maintained with approximately 3.5% isoflurane in O₂ by a face-mask. Body temperature was maintained throughout the surgical procedure by means of an electrically heated pad placed underneath each animal on the surgery table. Following surgical closure but prior to cessation of anesthesia, rats were hydrated with 10 ml of normal saline for injection, administered subcutaneously.

A small incision, (approximately 2 cm) was made on the ventral midline over the trachea. The right common carotid was exposed by blunt dissection. Exposure was accomplished to include the branch point of the internal and external carotid arteries from the common carotid artery and the branch point of the middle cerebral artery (MCA) from the internal carotid. Sutures (3-0 silk) were placed around the common and external carotid arteries and tied in place.

A 3-0 nylon suture filament, with a heat-formed rounded tip and coated at the tip with a very thin layer of silicon grease was inserted 18 to 20 mm up the exposed internal carotid and up the MCA to the junction of the circle of Willis and tied in place for 60 minutes. After 60 minutes, the filament was removed. The repair composition was administered using the hole in the internal carotid from which the filament had been removed, prior to reperfusion. Cyanoacrylate tissue cement was applied to seal the hole, the ties were removed from the external and common carotid arteries permitting reperfusion, and the wound was closed using wound clips.

Approximately 0.1 ml total fluid volume (containing a total of about 2.3 µg) of therapeutic composition (therapeutic treatment) or saline alone (sham treatment) was injected into the internal carotid, immediately upstream (2-3 mm) of the branch point of the MCA using a blunt, 25G cannula that had been inserted past the branch point such that its tip was located in the MCA. The cannula was tied in with 3-0 silk to prevent back-flow, and the composition or saline alone was administered over a period of approximately one minute. Approximately 5 minutes were allowed to elapse after injection of the therapeutic composition to allow for the association of the composition with the brain tissue, prior to reperfusion.

Experimental groups consisted of group 1 (control): rats (n=4) given sham treatment consisting of saline injection directly into the branch point of the middle cerebral artery (MCA) from the internal carotid artery after 1 hour of tMCAO and immediately prior to reperfusion; and group 2: rats (n=4) given therapeutic construct treatment injected directly into the branch point of the MCA from the internal carotid artery after 1 hour of tMCAO and immediately prior to reperfusion.

Anesthetic was discontinued, and following recovery of ambulation, each rat was tested for neurological deficit (contralateral paw flexion, unequal ocular response, and circling) to establish the presence of ischemia. The rats were then placed in single cages in the postoperative room for recovery and kept warm by heating lamps until the next day. Immediately prior to being humanely euthanized 6 days after MCAO, the animals of groups 1 and 2 were evaluated for neurological deficit.

**Table 2 Neurological Severity Scores (NSS)**

| **I. Motor Tests** | Item score | Total possible |
|---|---|---|
| **Raising rat by the tail** | | 3 |
| Flexion of forelimb | 1 | |
| Flexion of hind limb | | |
| Head moved 10° to vertical axis within 30 seconds | 1 | |
| **Placing rat on the floor (normal-0; maximum-3)** | | 3 |
| Normal walk | 0 | |
| Inability to walk straight | 1 | |
| Circling toward the paretic side | 2 | |
| Fall down to the paretic side | 3 | |

| **II. Sensory tests** | | |
|---|---|---|
| **Reflexes absent and abnormal movements (normal**- | | 4 |
| **0; maximum-4)** | | |
| Pinna reflex (head shake when touching the auditory | 1 | |
| meatus) | | |
| Corneal reflex (eye blink when lightly touching the | 1 | |
| cornea with cotton) | | |
| Startle reflex (motor response to a brief noise from | 1 | |
| snapping a clipboard paper) | | |
| Seizures, myoclonus, myodystony | 1 | |
| | | |
| **III. Ptosis** | | 3 |
| **(Normal-0; maximum-3)** | | |
| **MAXIMUM SCORE** | | **13** |

Table 2 shows a set of modified Neurological Severity Scores (NSS). Neurological function was graded on a scale of 0 to 10 (normal score, 0; maximal deficit score, 10). The modified NSS shown is a composite of motor and sensory tests. In the severity scores of injury, 0 score-point is awarded for a normal response and 1 score-point is awarded for the inability to perform the test or for the lack of a tested reflex; thus, the higher the combined score, the more severe the overall injury; 7 to 10 indicates severe injury; 3 to 6, moderate injury; 1 to 2, mild injury; 0, no injury (normal rats prior to surgery or sham surgery).

Animals were followed for 6 days after tMCAO, and at that time animals were humanely euthanized by CO₂ asphyxiation. After euthanasia, the brains were perfused transcardially and collected for histomorphologic analysis. In brief, rat brains were flushed of blood by transcardial perfusion with saline, and then fixed by perfusion with 100 ml of 10% neutral, buffered formalin. Each brain was then carefully dissected and removed from the cranium and immersed in 10% neutral buffered formalin. The brains were then trimmed of the thalamus embedded in paraffin. The cerebral tissues were cut into 3 equally-spaced coronal blocks, stained with hematoxylin and eosin, and sectioned into 12 µM sections for mounting onto glass slides. A series of adjacent sections were cut from each block in the coronal plane.

The results of experimental example 2 are now presented.

The results are summarized in Tables 3 and 4. Two of four control animals died within three days of stroke. None of the four treated animals died prior to euthanasia.

**Table 3 Collective results**

| Animal | Days post-op | n= | clubbing | drag | circles | eyes |
|---|---|---|---|---|---|---|
| Controls | 0 | 4 | 3 | 3 | 3 | 3 |
| | 6 | 2 | 2 | 1 | 2 | 3 |
| Treated | 0 | 4 | 3 | 3 | 3 | 3 |
| | 6 | 4 | 1 | 0 | 1 | 1 |

**Table 4 Results at Day 6 for individual rats administered repair composition 1**

| | clubbing | drag | circles | eyes |
|---|---|---|---|---|
| 1 | 1 | 0 | 1 | 2 |
| 2 | 1 | 0 | 0 | 2 |
| 3 | 0 | 0 | 0 | 0 |
| 4 | 1 | 0 | 1 | 1 |

Before occlusion, neither group of rats showed a difference in paw use as judged by visual observation of the limbs during normal ambulation on a flat surface. Immediately after surgery, both the control and treated rats showed marked paw disparity, with the left (contralateral) paw contracted in flexion significantly more often than the right, 5 indicative of contralateral sensorimotor dysfunction. This stable abnormality persisted throughout testing in all control tMCAO animals. In marked contrast, in rats receiving the therapeutic composition treatment as described above, the stroke-induced forepaw disparity was not significant by 6 days after transplantation with virtually no difference between the paws.

Baseline (spontaneous) rotation was significantly asymmetric in stroke (tMCAO) rats receiving the control treatment (saline injection). These rats, immediately following treatment, circled definitively to the right when placed on a flat surface and allowed to ambulate spontaneously, and did not entirely correct this behavior by day 6. Conversely, rats treated with therapeutic construct immediately following treatment circled definitively to the right when placed on a flat surface, but had by day 6 almost imperceptible, if any, circling, and several showed comparable turning in both directions.

In all cases, all animals subjected to tMCAO were observed to have unequal but reactive eyes. This inequality was the result of an inability of the rats to open the contralateral eye to its fullest, normal extent indicative of contralateral sensorimotor dysfunction. This stable abnormality persisted throughout day 6 in all control tMCAO animals. In general, most treated animals retained some inequality in their ocular presentation at day 6. However, one of the 4 treated animals, in addition to demonstrating no circles and no contralateral paw flexion, also had no evidence of ocular inequality 6 days following tMCAO.

A certified pathologist performed a histological review of cerebral sections for each animal. The histology findings on day 6 for each animal is now presented.

Control animal 1: A very minimal unilateral lesion was present at the level of the mid hippocampus and involves only the interior capsule. The lesion was a small focus of gliosis and vacuolation of the white matter. This level was the caudal most level examined. The other sections of brain were all rostral to this section and contained no evidence of infarction.

Control animal 2: The infarct was evident on most sections but was most severe at the level of the anterior hippocampus. The lesion at that level was mostly confined to the basal ganglia unilaterally and was composed of areas of necrosis and gitter cells within the basal ganglia, edge of the hypothalamus and internal and external capsules. The cortex was affected, mostly ventrally, and the lesions were mostly areas of neuronal necrosis within the middle and/or deep neuronal layers (layers 3-6). The hippocampus was unaffected and the thalamus minimally affected. The rostral sections contained lesions in the basal ganglia (gitter cells and neuronal necrosis) and scattered neuronal necrosis in the cortex. The caudal most section, at the level of the aqueduct, contained areas of necrosis in the ventral cortex, with scattered individual necrotic neurons in the middle and/or deep neuronal layers in the lateral brain.

Treated animal 1: There was no microscopic evidence of infarction in any of the sections.

Treated animal 2: There was no microscopic evidence of infarction in any of the sections.

Treated animal 3: There was no microscopic evidence of infarction in any of the sections.

Treated animal 4: There was no microscopic evidence of infarction in any of the sections.

Upon histological review by a certified pathologist, evidence of infarction was present in the control animals, validating the technique used to create the lesion. Infarcted areas were unilateral and generally most severe at the level of the anterior hippocampus.

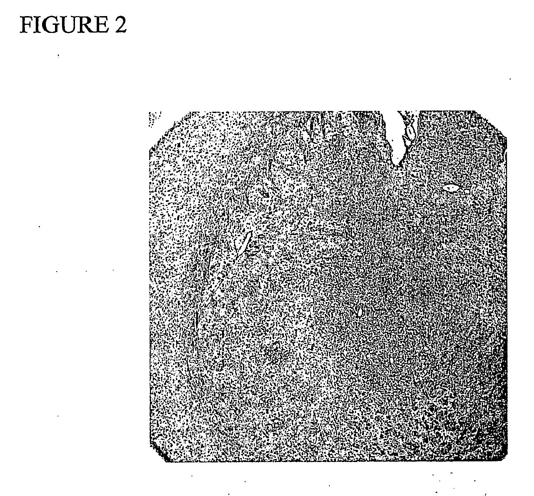

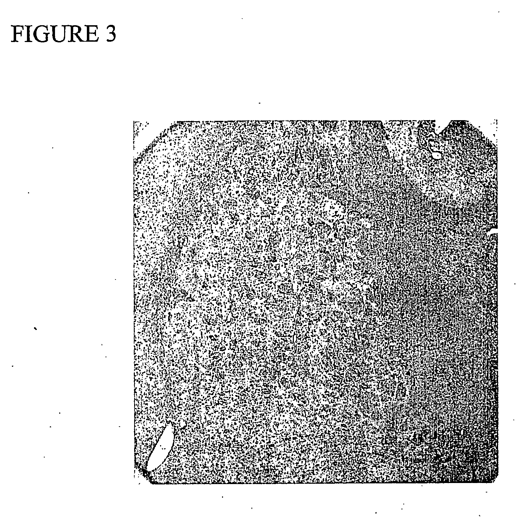

Figure 2, a representative histological section from the brain of control animal 2, shows evidence of infarction and necrosis consistent with ischemic stroke. The treated animals, however, did not show detectable histomorphologic evidence of infarction. Figure 3 is a representative histological section from a section of the brain of treated animal 4. These data support the efficacy of the composition in treating reperfusion injury.

### Experimental Example 3

The following experiments were designed to assess the effects of compositions of the invention in a more severe acute ischemic stroke model than in Experimental Example 2. In this stroke model, the middle cerebral artery was occluded for 2 hours.

The materials and methods used in the experiments presented in this Example are now described.

Repair composition 1, comprising anti-CD54 antibody, anti-CD90 antibody and EGF, was prepared as follows. Ten (10) µg biotinylated anti-CD54 antibody, 10 µg biotinylated anti-CD90 antibody and 5 µg biotinylated EGF (molar ratio of 1:1:2) were mixed together, added to 20 µg avidin in a total volume of 400 µl, and allowed to bind for 30 minutes at 22 °C. The repair composition solution was then diluted to 800 µl with PBS.

Repair composition 2, comprising anti-CD54 antibody and anti-CD90 antibody, was prepared as follows. Twenty (20) µg biotinylated anti-CD54 antibody and 20 µg biotinylated anti-CD90 antibody (molar ratio 1:1) were mixed together, added to 20 µg avidin in a total volume of 400 µl, and allowed to bind for 30 minutes at 22 °C. The repair composition solution was then diluted to 800 µl with PBS.

Per an IACCUC-approved protocol, 3 groups of rats (6 week old Sprague Dawley males) were anesthetized, and subjected to carotid incision, and right middle cerebral artery occlusion for 2 hours. At the time of reperfusion and in a blinded manner, 150 µl of a repair composition or saline (control) was delivered into the carotid by the veterinary surgeon. Two rats received saline (animals 102 and 202), one rat received Repair Composition 1 (animal 101) and another rat received Repair Composition 2 (animal 201). The incision was closed, and the animals returned to their cages. Under blind conditions, animals were scored by an investigator for neurological deficits three times: upon recovery from anesthesia (post-surgery), at 24 hours after surgery and at day 6 (prior to sacrifice). Control rat 102 died before 24 hours had elapsed from time of treatment.

At day 6, after the evaluation of neurological deficit, the animals were sacrificed, and the brains removed for histological analysis.

The neurological results for the three surviving animals were as follows. Animal 101 had minimum residual injury. Animal 201 also had minimum residual injury. In sharp contrast, animal 202, which received saline, had severe neurological damage.

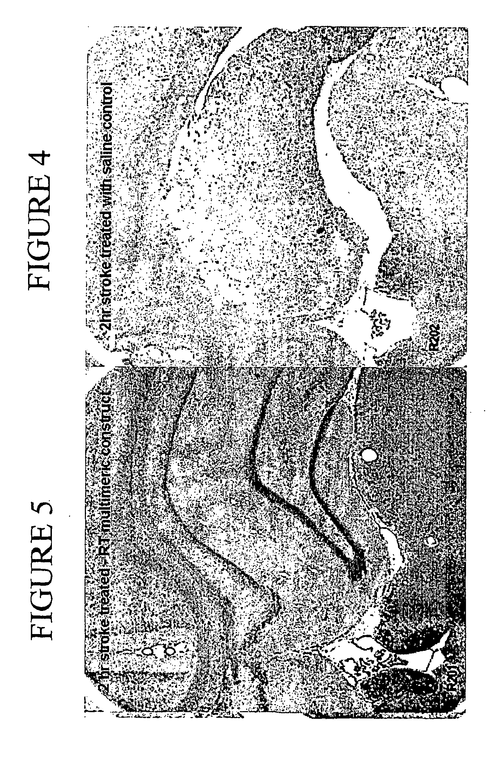

The histological sections from the control had severe necrosis. The histological sections from the treated animals, however, were nearly normal. Images of representative histological sections from the control (animal 202) and an animal treated with Repair Composition 2 (animal 201) are shown in Figures 4 and 5, respectively.

### Experimental Example 4

The following experiments were performed using the one hour occlusion stroke model used in Experimental Example 2.

The materials and methods used in the experiments presented in this Example are now described.

Repair composition 1, comprising anti-CD54 antibody, anti-CD90 antibody and EGF, was prepared as follows. Ten (10) µg biotinylated anti-CD54 antibody, 10 µg biotinylated anti-CD90 antibody and 5 µg biotinylated EGF were mixed together, added to 20 µg avidin in a total volume of 400 µl, and allowed to bind for 30 minutes at 22 °C. The repair composition solution was then diluted to 800 µl with PBS. This composition was prepared one week in advance of the experiment and stored in a refrigerator.

Repair composition 2, comprising an anti-CD54 antibody and a anti-CD90 antibody, was prepared as follows. Twenty (20) µg biotinylated anti-CD54 antibody and 20 µg biotinylated anti-CD90 antibody were mixed together, added to 20 µg avidin in a total volume of 400 µl, and allowed to bind for 30 minutes at 22 °C. The repair composition solution was then diluted to 800 µl with PBS. This composition was prepared on the same day as it was administered.

Per an IACCUC-approved protocol, 3 groups of 5 rats (6 week old Sprague Dawley males) underwent anesthesia, and were subjected to carotid incision, and right middle cerebral artery occlusion for 2 hours. At the time of reperfusion, 150 µl of a repair composition or saline (control) was delivered into the carotid in a blinded manner by the veterinary surgeon. The incision was closed and the animals returned to their cages. Under blind conditions, animals were scored by an investigator for neurological deficits three times: upon recovery from anesthesia (post-surgery), at 24 hours after surgery and at day 6 (prior to sacrifice). At 6 days, after the neurological evaluation, animals were humanely sacrificed and the brains were removed for histological analysis. H&E and cresyl violet stained coronal sections of brain were examined microscopically. The histological analysis was performed by a certified pathologist.

The results of the experiments are now presented.

Of the 15 rats, one rat died during the occlusion procedure, and six rats died 24 hours or less after the procedure (plus or minus treatment). The death during the occlusion procedure was attributed to a torn common carotid. The remaining 6 deaths were attributed to the model. Two control mice died, four rats that were treated with Repair Composition 1 died and one rat treated with Repair Composition 2 died. Survivors are summarized in Table 5. This model commonly has a reported mortality of about 50% in the literature. Experimental Example 2 indicated a survival benefit for rats treated with Repair Composition 1 compared to those treated with saline. A similar benefit was not observed for Repair Composition 1 in these experiments. It is thought that the quality of the repair composition was compromised, possibly by the week of storage between preparing the composition and using it.

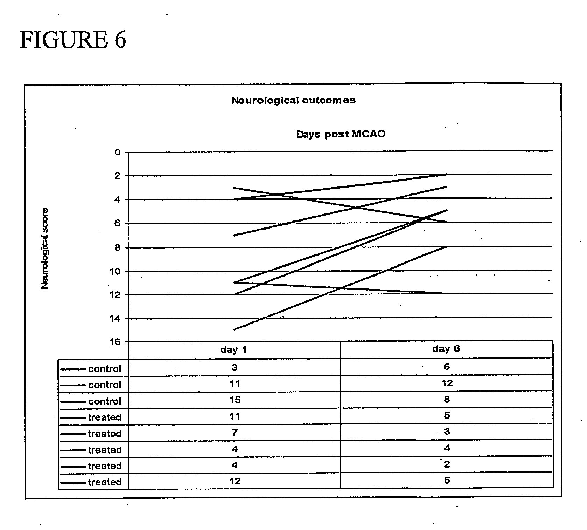

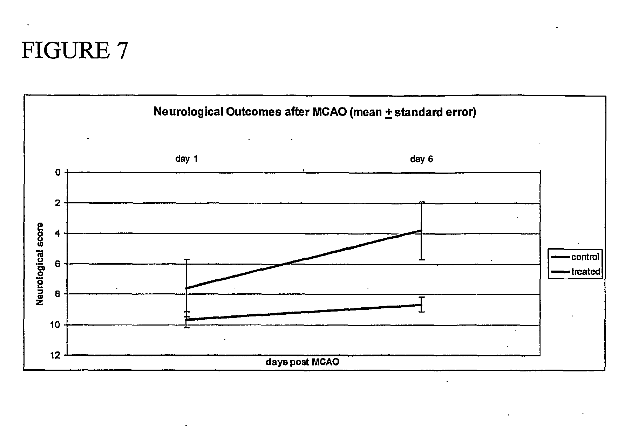

All of the treated animals experienced an improvement in neurological outcome, while two of the three control rats did not improve. Scores for individual animals are shown in Figure 6, which also depicts the data graphical. Cumulative scores for the treated rats were better at both day 1 and day 6, compared to control rats (Figure 7).

**Table 5**

| **Animal No.** | **Treatment** |
|---|---|
| 3B202 | saline |
| 3B502 | saline |
| 3B102 | Anti-ICAM + Anti-CD90 + EGF (Repair Composition 1) |
| 3B201 | Anti-ICAM + Anti-CD90 (Repair Composition 2) |
| 3B501 | Anti-ICAM + Anti-CD90 (Repair Composition 2) |
| 3B602 | Anti-ICAM + Anti-CD90 (Repair Composition 2) |
| 3B801 | Anti-ICAM + Anti-CD90 (Repair Composition 2) |
| 3B401 | Unknown* |

| | |
|---|---|
| * A technician initially listed this an "unknown". Based on initial blinded histology evaluation, this animal was analyzed as a control. | |

For the histological analysis, sixteen transverse sections representing four levels of the brain were examined from each animal. Eight sections were stained with H&E, and eight sections were stained with cresyl violet stain. The levels were somewhat consistent between animals. The rostral-most section usually contained the intrabulbar part of the anterior commissure and anterior olfactory nucleus. The second section was near bregma and contained caudate putamen, nucleus accumbens and corpus callosum. The third section was at the level of the anterior hippocampus and contained thalamus and hypothalamus, and the caudal-most section was usually at the midbrain (cerebral aqueduct) and contained the substantia nigra and medial geniculate nucleus.

### Saline Controls

Rat #3B202: An infarct was present and was observed in all four sections of brain; subgrossly, the affected area of brain lacked staining intensity in both H&E and Nissl stains. The area of pannecrosis and cavitation extended unilaterally from the insular cortices at the level of the intrabulbar part of the anterior commissure, caudally to the rostral hippocampus and thalamus. The infarcted area was largest and most severe at the level of the optic chiasm; infarcted tissue was much less in the other three levels of brain examined. Pannecrosis, involving all cell types, unilaterally involved the secondary somatosensory, insular and portions of the piriform cortex, amygdalo-piriform transition area, external capsule, lateral caudate putamen and an area of the hypothalamus. This core area consisted of parenchymal and cellular dropout and necrosis of the entire neuropil, with infiltration of gitter cells. There were no completely acellular areas. The adjacent parenchyma in the ipsilateral substantia nigra and somatosensory cortex showed gliosis and selective neuronal necrosis. The hippocampus, dentate gyrus and thalamus were largely spared. Minimal inflammation (mostly mononuclear when present) but no hemorrhage was observed.

Rat #3B502: A large infarct was present and was observed in all four sections of brain; subgrossly, one half of the brain was shrunken and lacked staining intensity in both H&E and Nissl stains. The area of pannecrosis and cavitation extended unilaterally from the frontal association and orbital cortices at the level of the intrabulbar part of the anterior commissure, caudally to medial geniculate nucleus at the level of the cerebral aqueduct. The infarcted area was largest and most severe at the level of the optic chiasm. Pannecrosis, involving all cell types, extended the entire height of the brain and unilaterally involved the entire cerebral cortex (frontal association, orbital, cingulate, motor, somatosensory, insular, piriform, auditory, visual, ecto- and perirhinal cortices and portions of the entorhinal cortex), olfactory tubercle, hypothalamus, caudate putamen and corpus callosum, reaching medially to the ipsilateral lateral ventricle. This core area consisted of a large area with no living tissue surrounded by an area of cellular necrosis with infiltration of glia and gitter cells. The ipsilateral retrosplenial and cingulate cortices were spared. The adjacent parenchyma in the thalamus, hypothalamus, substantia nigra and hippocampus showed gliosis and selective neuronal necrosis. Selective neuronal necrosis of the ipsilateral and contralateral sides usually involved neurons of layers 3 and 4, with sporadic involvement of other layers. Minimal inflammatory infiltrates (mostly mononuclear) were present ventrally, but no hemorrhage was observed.

Rat #3B401 ("Unknown treatment"): This animal appeared to have the largest infarct of the animals examined. The infarcted area included the frontal association and orbital cortices at its rostral-most point (at the level of the intrabulbar anterior commissure) and most of the major cerebral cortices at the level of the subcommissural organ. The core area of infarction, comprised of large acellular areas of necrosis and dropout of all cellular components surrounded by necrotic areas showing gliosis and gitter cell infiltration, included the thalamus, hypothalamus, caudate putamen, corpus callosum, olfactory tubercle, optic tract and portions of the hippocampus besides the major cortices already mentioned. Pannecrosis extended to medially to the edge of the lateral ventricle. Selective neuronal necrosis was observed in the medial geniculate nucleus and motor cortex. The retrosplenial and cingulate cortices were spared.

### Repair Composition 1 (anti-CD54 and anti-CD90 antibodies and EGF)

Rat #3B102: A large infarct was present and could be observed in all four sections of brain; subgrossly, one half of the brain was shrunken and lacked staining intensity in both H&E and Nissl stains. The area of pannecrosis and cavitation extended unilaterally from the level of the optic chiasm (which is the rostral-most section in this animal), caudally to medial geniculate nucleus at the level of the cerebral aqueduct. The infarcted area was largest and most severe at the level of the optic chiasm. Pannecrosis, involving all cell types, extended the entire height of the brain and unilaterally involved the entire cerebral cortex (orbital, primary motor, somatosensory, insular, piriform, auditory, visual, ectorhinal, entorhinal and perirhinal cortices), caudate putamen, corpus callosum, lateral preoptic area, ventral pallidum and olfactory tubercle. This core area of the infarct consisted of small, essentially acellular areas surrounded by areas of parenchymal and cellular dropout and necrosis of the entire neuropil; infiltration of gitter cells and some mononuclear cells, primarily in the ventral areas were noted. The adjacent parenchyma showed gliosis and selective neuronal necrosis, particularly in the lateral and dorsal thalamus. The ipsilateral retrosplenial and cingulate cortices, hypothalamus, hippocampus, dentate gyrus, substantia nigra and medio-ventril thalamus were spared. No hemorrhage was observed. A large organizing thrombus was present within a large artery at the base of the brain on the same side as the infarct.

### Repair Composition 2 (comprising anti-CD54 and anti-CD90 antibodies)

Rat #3B201: A large necrotic area similar in rostro-caudal size to that in rat #3B502 was observed. The infarcted area could be observed in all four sections of brain; subgrossly, one half of the brain was shrunken and lacked staining intensity in both H&E and Nissl stains. The area of pannecrosis extended unilaterally within the cerebral cortices at the level of the intrabulbar part of the anterior commissure, caudally to the level of the cerebral aqueduct. The infarcted area was largest and most severe at the level of the optic chiasm. Pannecrosis, involving all cell types, extended the entire height of the brain and unilaterally involves the entire cerebral cortex (frontal association, orbital, cingulate, motor, somatosensory, insular, piriform, auditory, visual, ecto- and perirhinal cortices and portions of the entorhinal cortex) except for the retrosplenial and cingulate cortices. The core area in the cortex consisted of parenchymal and cellular dropout and necrosis of the entire neuropil, with infiltration of gitter cells. There were no completely acellular areas. Lesser affected areas, such as the caudate putamen and corpus callosum, contained ghost nuclei, gliosis and vacuolation of the neuropil. The ipsilateral retrosplenial and cingulate cortices, hippocampus, dentate gyrus, substantia nigra, thalamus and hypothalamus were spared. Minimal inflammation (mostly mononuclear when present) but no hemorrhage was observed.

Rat #3B501: An area of infarction was present within the basal ganglia unilaterally and consisted of an acellular focus of necrotic neuropil surrounded by an area containing ghost nuclei and gliosis with occasional vacuolation. There were no foci of neuropil dropout. The infarcted area was limited to an area of the medial caudate putamen, nucleus accumbens and anterior commissure immediately adjacent to the lateral ventricle. One focus of vacuolated neuropil and gliosis was within the ipsilateral internal capsule. No hemorrhage was observed.

Rat #3B602: A large infarcted area similar in size and appearance to rat #3B201 was present. The infarcted area in the cerebral cortices extended from the level of the intrabulbar portion of the anterior commissure, caudally to the level of the midbrain. The cerebral cortices except for the retrosplenial, cingulate and visual were affected, as well as a good portion of the ipsilateral corpus callosum and caudate putamen; the area of involvement consisted of parenchymal and neuronal necrosis and dropout with cavitation and infiltration of gitter and glial cells. There were no completely acellular areas. Selective neuronal necrosis was also observed in the CA1 field of the ipsilateral hippocampus at the level of the thalamus, and gliosis was observed in the ipsilateral globus pallidus and the thalamus at the level of the subcommissural organ. A large organizing thrombus was present within a large artery at the base of the brain on the same side as the infarct, and hemorrhage was observed in the meninges.

Rat #3B801: There was no evidence of infarction in any of the sections examined.

The evidence of infarction present in the control animals validated the technique used to create the lesion. The infarcted areas varied in appearance and size in these animals. The affected areas of brain in the Repair Composition 1- treated animals with the large infarctions were similar to those affected in control animal #3B502; however, there were some differences in the microscopic appearance of the core. In the three Repair Composition 1- treated animals with microscopic infarctions, the necrotic zones consisted of areas undergoing resolution, as evidenced by the presence of glia and gitter cells, generally in large numbers and generally throughout the core. Small areas of complete acellularity were observed only in animal #3B501. In comparison, control animal #3B502, which had a large area of infarction, showed gitter cells and gliosis primarily at the edge of the core, with some penetration into the central necrotic zone. This difference in the amount of cellular response to injury may be attributable to repair composition treatment, although the number of animals examined is small.

Animal #3B102, treated with Repair Composition 2, showed a large core of infarction with acellular areas and gliosis/gitter cell infiltrates, most similar to the saline controls. The significance of this cannot be determined, since this was the only animal in this treatment group that was examined microscopically. Additionally, the repair composition for this animal was not freshly prepared prior to administration.

Animal #3B401 that was indicated as having an "unknown" treatment post surgically was most similar to control animal #3B502, in that the core of infarction showed acellular areas with gliosis and gitter cells mostly at the edges of the infarcted area.

Thus, Repair Compositions of the invention, with or without growth factor, demonstrated improvement in neurological function and outcome. Histological analysis correlated increased cellular remodeling with the treatment constructs.

### Experimental Example 5

Acute renal failure after renal stenosis has been shown to have both tubular destruction and inflammatory components.

The following experiments were designed to assess whether compositions of the invention had activity and specificity in the setting of renal occlusion.

The materials and methods used in the experiments presented in this Example are now described.

Repair composition 2, comprising anti-CD54 antibody and anti-CD90 antibody, was prepared as follows. Ten (10) µg anti-CD54 antibody and 10 µg anti-CD90 antibody were mixed together, added to 10 µg avidin in a total volume of 400 µl, and allowed to bind for 30 minutes at 22 °C. The repair composition solution was then diluted to 800 µl.

Repair composition 3, comprising an anti-CD105 antibody and a anti-CD90 antibody, was prepared as follows. Ten (10) µg anti-CD105 antibody and 10 µg anti-CD90 antibody were mixed together, added to 10 µg avidin in a total volume of 200 µl, and allowed to bind for 30 minutes at 22 °C. The repair composition solution was then diluted to 800 µl.

Per an IACCUC-approved protocol, 3 groups of male Sprague Dawley rats (n=5 per group) underwent anesthesia, and were subjected to abdominal incision and left renal artery occlusion with the use of a micro-vascular clamp for 45 minutes. A skin incision was also made over the left carotid artery, and a catheter was passed from the carotid to the aorta. After the renal artery clamp was removed, and blood flow reestablished into the kidney, 150 ul of either Repair Composition 2, Repair Composition 3 or PBS (as a control) was delivered into the aortic catheter in a blinded manner by the veterinary surgeon. The incisions were then closed, and the animals allowed to recover from anesthesia, and returned to their cages. One animal, which had received Repair Composition 2, died from a rupture of the carotid artery prior to the closure of the skin incisions. This treatment group (treatment with Repair Composition 2) therefore had only 4 rats in it.

) At 7 days post-occlusion and treatment, the animals were sacrificed, and the left kidney removed for histology. The histology sections were scored according to the tubular damage or inflammation (Table 6).

**Table 6 Histology Scoring System**

| Tubular Dilatation | | Inflammation | |
|---|---|---|---|
| None | 0 | None | 0 |
| Mild focal | 1 | Mild | 1 |
| Mild | 2 | Moderate | 2 |
| Moderate | 3 | Severe | 3 |
| Severe | 4 | | |

The results of the experiments described in the example are now presented.

The repair compositions used in this example were designed to bind to kidney endothelial cells or injured kidney cells and injured kidney endothelial cells, and to a repair cell. CD 105 is expressed on endothelial cells in the kidney. CD54 (ICAM-1) is expressed on injured kidney cells and injured kidney endothelial cells. CD54 is more highly expressed than CD105 in inflammatory injury. CD 90 is expressed on repair and progenitor cells.

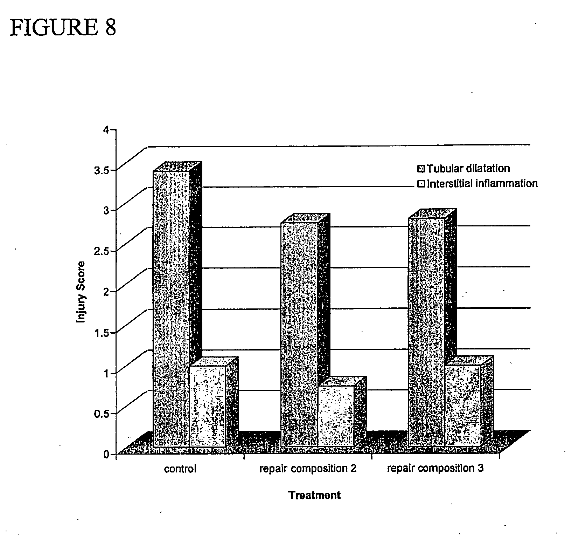

The average results for the three groups are summarized in Table 7. Figure 8 depicts these data graphically. The results for individual subjects in each group are summarized in Table 8.

**Table 7**

| **Mean Scores** | **Tubular Dilatation** | **Inflammation** |
|---|---|---|
| Control (PBS) | 3.4 | 1 |
| Treatment with Repair Composition 2 | 2.75 | 0.75 |
| Treatment with Repair Composition 3 | 2.8 | 1 |

**Table 8**

| **Animal number** | **Tubular dilatation** | **Score** | **Interstitial inflammation** | **Score** |
|---|---|---|---|---|
| **Control** | | | | |
| 101 | Moderate | 3 | mild | 1 |
| 104 | Moderate | 3 | mild | 1 |
| 106 | Severe | 4 | mild | 1 |
| 113 | Severe | 4 | mild | 1 |
| 115 | Moderate | 3 | mild | 1 |
| | | **3.4** | | **1** |

| Repair Composition 2 | | | | |
|---|---|---|---|---|
| 103 | Mild | 2 | none | 0 |
| 108 | severe | 4 | moderate | 2 |
| 110 | focal mild | 1 | none | 0 |
| 112 | severe | 4 | mild | 1 |
| | | **2.75** | | **0.75** |

| Repair Composition 3 | | | | |
|---|---|---|---|---|
| 109 | severe | 4 | moderate | 2 |
| 111 | mild | 2 | mild | 1 |
| 114 | focal mild | 1 | none | 0 |
| 102 | severe | 4 | mild | 1 |
| 105 | moderate | 3 | mild | 1 |
| | | **2.8** | | **1** |

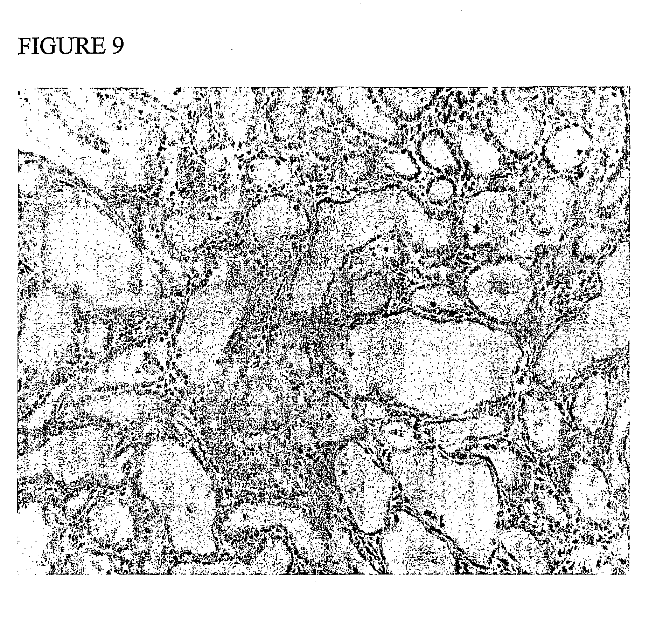

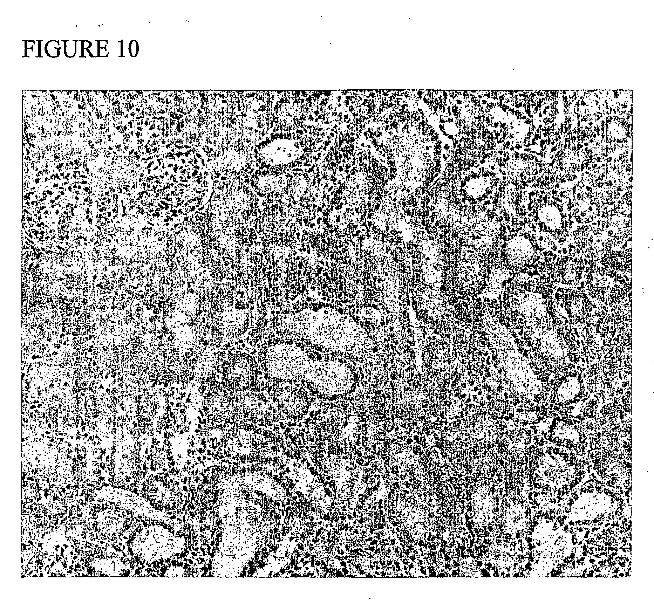

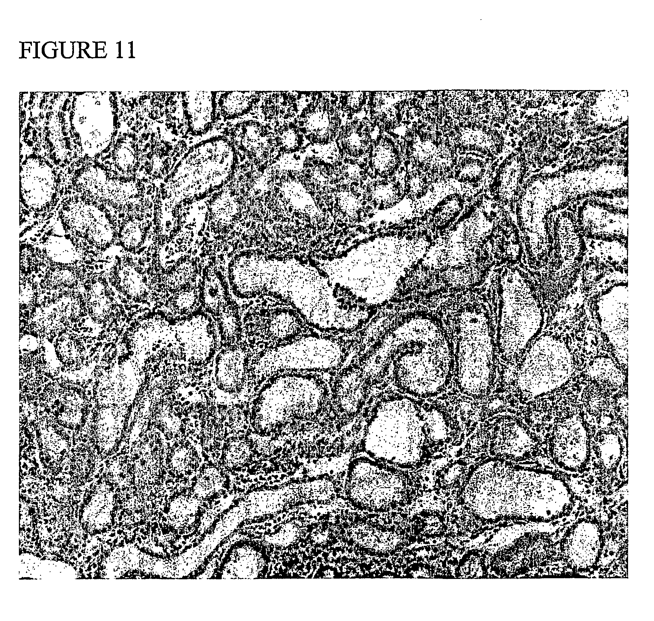

Figures 9, 10 and 11 depict images of representative brain sections from animals treated with saline, Repair Composition 2 and Repair Composition 3, respectively. In control animal 106, severe tubular dilatation with mild chronic inflammation is evident (Figure 9). In animal 103, treated with Repair Composition 2, only mild focal tubular dilatation is observed (Figure 10). In animal 105, treated with Repair Composition 3, moderate tubular dilatation with mild chronic inflammation is evident (Figure 11).

Thus, both Repair Composition 2, comprising an anti-CD54 antibody and a anti-CD90 antibody and Repair Composition 3, comprising an anti-CD105 antibody and a anti-CD90 antibody, reduced the tubular damage in kidney cells subjected to renal stenosis, compared to controls. Composition 2 was observed to have a greater effect on reducing inflammation than Composition 3 (Table 7).

### Experimental Example 6

Normal CD45⁺ lymphocytes have been reported in the literature to bind only injured aorta, e.g., atherosclerotic injured aorta, but not normal, uninjured aorta. CD61 (integrin β₃ / vitronectin) and CD106 (VCAM-1) are both expressed on normal aortic endothelium.

The following experiments were designed to assess whether compositions of the invention could successfully bind normal CD45⁺ lymphocytes to normal, uninjured aorta tissue.

The materials and methods used in the experiments presented in this Example are now described.

Bone marrow was obtained from two femurs of male Sprague Dawley rats, suspended in PBS, layered onto 15 ml Ficoll-Hypaque (specific gravity 1.072) and centrifuged for 20 minutes at 400g. Mononuclear cells were collected from the interface of the layers, washed and plated at 37°C for 45 minutes to deplete monocytes. Half of the resulting lymphocytes were cultured in 25 microgram DiI fluorescent membrane marker at 37 °C for 30 minutes. The DiI-labeled cells were washed and resuspended in PBS.

Normal aorta was removed from a male Sprague Dawley rat, taking care to keep tissue free from vascular injury. The aorta was carefully sliced into 5 mm sections and maintained ex vivo for 30 minutes in lactated Ringer's solution on ice until the lymphocytes were ready.

Repair Composition 4, comprising an anti-CD45 antibody and a anti-CD61 antibody, was prepared as follows. Ten (10) µg biotinylated anti-CD61 antibody and 10 µg biotinylated anti-CD45 antibody were mixed together, added to 10 µg avidin in a total volume of 200 µl PBS, and allowed to bind for 30 minutes at 22 °C. The repair composition was diluted with PBS to a final volume of 500 µl.

Repair Composition 5 was prepared as follows. Ten (10) µg anti-CD106 antibody and 10 µg anti-CD45 antibody were mixed together, added to 10 µg avidin in a total volume of 200 µl, and allowed to bind for 30 minutes at 22 °C. The repair composition was diluted with PBS to a final volume of 500 µl.

Fifty (50) µl of each repair composition was placed onto an aorta section and allowed to react for 30 minutes at 37°C. A control aorta section had 50 µl of PBS and was incubated under the same conditions. After the incubation, sections were washed with PBS and 1x10⁵ DiI-labeled lymphocytes were added to each section. The sections were then returned to the 37°C incubator for an additional 30 minutes. The aortic sections were then rinsed with PBS and viewed under a fluorescent microscope.

The results of the experiments are now presented.

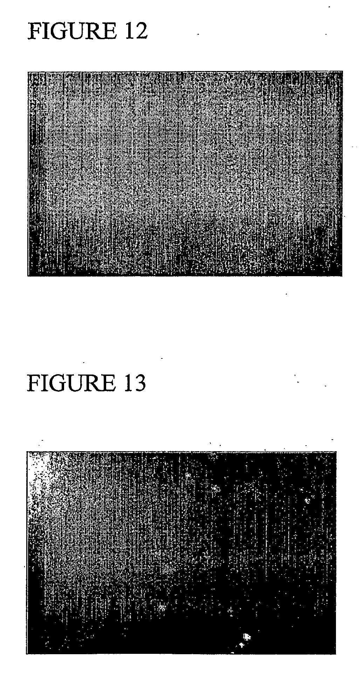

DiI-labeled lymphocytes did not bind to the PBS-treated aorta section (Figure 12). This result was expected, based on the knowledge in the art.

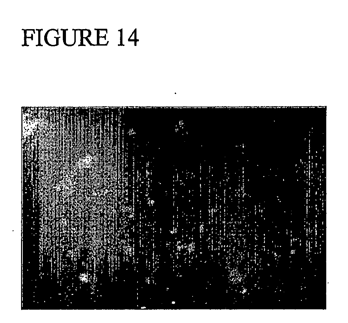

In contrast, a moderate amount of DiI-labeled lymphocytes bound to Repair Composition 4, comprising anti-CD61 and anti-CD45 antibodies (Figure 13). Notably, a large amount of Dil-labeled lymphocytes bound to Repair Composition 5, comprising anti-CD 106 and anti-CD45 (Figure 14).

Thus the repair compositions of the invention were capable of mediating a non-physiological interaction: normal CD45⁺ lymphocytes bound to normal, uninjured aortic tissue. In the absence of a repair composition of the invention, lymphocytes were not detectably-bound to aortic tissue. However, both repair compositions of the invention enabled detectable lymphocyte binding to normal aortic tissue. The repair composition comprising anti-CD106 provided a greater amount of lymphocytes to be bound compared to the repair composition comprising anti-CD61. This result is consistent with the known expression levels of CD61 and CD106 on aortic endothelium; CD61 (integrin β₃ / vitronectin) is moderately expressed and CD106 (VCAM-1) is highly expressed.

### Experimental Example 7

Reperfusion injury has been demonstrated after cardiac angioplasty and stent placement. The following experiment was designed to assess binding activity of a repair composition of the invention in the setting of coronary.

The materials and methods used in the experiment presented in this Example are now described.

Repair Composition 2, comprising an anti-CD54 antibody and a anti-CD90 antibody, was prepared as follows. Ten (10) µg biotinylated anti-CD54 antibody and 10 µg biotinylated anti-CD90 antibody were mixed together, added to 10 µg fluorescein-labeled avidin in a total volume of 400 µl, and allowed to bind for 30 minutes at 22 °C. The repair composition solution was then diluted to 600 µl with PBS.

Per an IACCUC-approved protocol, male Sprague Dawley rats were anesthetized, subjected to thoracic incision, and the heart exposed. The left anterior descending (LAD) artery was identified and ligated for 30 minutes and then reperfused for 10 minutes. Two hundred (200) µl of the repair composition was introduced into the LAD artery, and the animals were sacrificed shortly thereafter.

The LAD artery was dissected from the heart, sectioned lengthwise and evaluated under the fluorescent microscope to determine binding of the construct to the coronary vasculature.

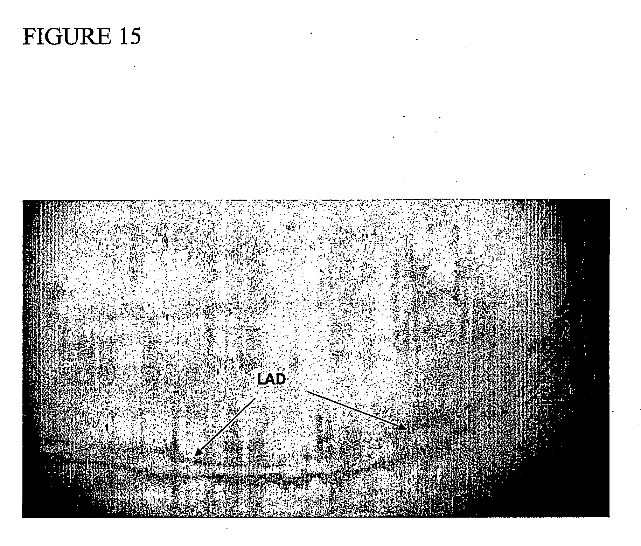

Fluorescence was detected in the LAD artery section, indicating that the repair composition successfully bound to the coronary vasculature (Figure 15).

### Experimental Example 8

Reperfusion injury has been demonstrated after femoral artery occlusion and stent placement. The following experiment was designed to assess the binding activity of a repair composition of the invention in the setting of peripheral vascular injury.

The materials and methods used in the experiment presented in this Example are now described.

Repair Composition 2, comprising anti-CD54 antibody and anti-CD90 antibody, was prepared as follows. Ten (10) µg biotinylated anti-CD54 antibody and 10 µg biotinylated anti-CD90 antibody were mixed together, added to 10 µg fluorescein-labeled avidin in a total volume of 400 µl, and allowed to bind for 30 minutes at 22 °C. The repair composition solution was then diluted to 600 µl with PBS.

Per an IACCUC-approved protocol, male Sprague Dawley rats were anesthetized, subjected to left inguinal incision and the femoral artery exposed. The femoral was catheterized and ligated for 1 hour and then reperfused for 15 minutes. 200 µl of the repair composition was introduced into the femoral, and the animal were sacrificed shortly thereafter.

The femoral artery was dissected out, removed and sectioned in half lengthwise, opened with the internal vascular surface exposed and evaluated under the fluorescent microscope to determine binding of the construct to peripheral vasculature.

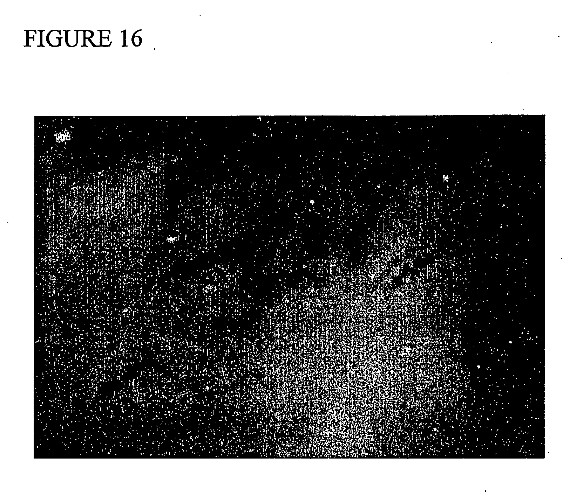

Fluorescence was detected in the femoral artery section, indicating that the repair composition successfully bound to the peripheral vasculature (Figure 16).

### Experimental Example 9

A patient presents with symptoms of stroke and is preferably evaluated clinically and with radiographic (CT scan or MRI) or angiographic procedures. The repair composition (Repair Composition 1) to be administered to this patient comprises: 1) a biotinylated anti-CD54 (ICAM-1) monoclonal antibody as the anchor moiety; 2) a biotinylated anti-CD90 (Thy-1) monoclonal antibody as the hook moiety; and 3) avidin. The repair construct is delivered intra-arterially, conveniently through a radiographic angiocath if present, or through an intra-arterial line. Where the stroke is due to a blockage of the carotid artery, the physician may perform an atherectomy or place a carotid stent (Guidant Corp.) and infuse the repair composition directly into the carotid artery. The repair composition is administered at least one hour after stroke in a pharmaceutically acceptable carrier. Repair construct administration may follow, precede or be contemporaneous with an optional thrombolytic reperfusion procedure (TNKase®, Genentech, South San Francisco, CA).

### Experimental Example 10

A patient presents with symptoms of a connective tissue injury and is evaluated clinically and with radiographic (CT scan or MRI) procedures. The repair composition (Repair Composition 6) to be administered to this patient comprises: 1) a anti-CD54 (ICAM-1) monoclonal antibody as the anchor moiety; 2) a anti-CD105 monoclonal antibody as the hook moiety; and 3) TGFbeta. The repair composition is absorbed onto a resorbable mesh of polyglactin 910 (Vicryl^{®}, Ethicon) as a matrix, although the repair composition can be covalently bonded to the resorbable mesh as an alternative.

In the treatment of acute articular cartilage injury or acute meniscal injury, the composition is press fit into place in the damaged tissue during arthroplasty (open surgical procedure). Alternatively, the construct is implanted during arthroscopy (closed surgical procedure). Tissue sealants, such as fibrin glues (Tisseal^{®}, Baxter, Deerfield, IL) or bioabsorbable sutures or darts (Ethicon), may be employed to help secure the construct in place.

### Experimental Example 11

A patient suffering from a traumatic skin injury is treated with a repair composition comprising an anchor moiety which target alpha integrins and an anti-CD44 hook which targets skin RCs. In one embodiment (Repair Composition 7), the anchor comprises a moiety that binds alpha V (CD51) and a moiety that binds alpha 5 (CD49e) and an anti-CD44 antibody as the hook. In another embodiment, the repair composition does not comprise a moiety that binds alpha 5 (CD49e). The repair composition is bound to a resorbable Vicryl^{®} matrix mesh.

The repair composition is implanted into or onto the injured epidermal and/or dermal layer of the skin and press fit into place. Tissue sealants, such as fibrin glues (Tisseal^{®}) or bioabsorbable sutures (Ethicon), or adhesive dressings are employed to help fix the construct in place. The repair composition is used in either autologous or allogeneic skin grafting.

### Experimental Example 12

A myocardial ischemia patient is evaluated clinically and receives therapy, preferably thrombolytics (TNKase^{®}) or percutaneous transluminal coronary angioplasty (PTCA), to reestablish patency of occluded blood vessels. A stent may be placed as part of the therapeutic procedure (Cypher®, Cordis Corporation, Miami Lakes, FL; Taxus®, Boston Scientific, Natick, MA). This is accompanied by intracoronary delivery of the repair composition through the coronary catheter delivery device (Guidant). An alternative delivery route is through intravenous infusion or direct coronary tissue injection. As ICAM-1 is upregulated on the surface of endothelial cells lining the blood vessels after vascular injury, the anchor moiety is an antibody specific for ICAM-1. The hook is an anti-CD90 antibody. CD90 is present on RCs originating from the marrow, neural progenitors and hematopoietic stem cells, as well as ADAS cells originating in adipose tissue. The repair composition (Repair Composition 8) further comprises VEGF, which stimulates growth and differentiation of angiogenic progenitors, as a signaling factor. The moieties are noncovalently bound to a biocompatible material.

### Experimental Example 13

A patient presents with spinal cord injury and the clinician may choose to perform surgical procedures to decompress the spinal cord. Within four weeks of the injury, a repair composition of the invention is administered to the patient locally to the spinal cord at the site of injury or surrounding tissue, or distant to the injury via delivery to the cerebrospinal fluid. If a cyst develops in the spinal cord subsequent to the injury, the construct is directly delivered into and around the cyst.

The repair composition (Repair Composition 9) comprises a moiety that binds N-CAM (CD56) as the anchor moiety, a moiety that binds CD90 and FGF as a signaling factor. An alternative composition that can be administered comprises two anchor moieties: a moiety that binds N-CAM (CD56) and a moiety that binds I-CAM-1 (CD54). The hook moiety is a moiety that binds CD90 and the signaling factor is FGF. In one aspect, the moieties of the composition are noncovalently bound to avidin. In another aspect, useful for space filling applications, as in the case of cysts, the moieties of the composition are covalently or noncovalently bound to a biocompatible mesh such as Vicryl^{®}.

### Experimental Example 14

A cardiac donor becomes available for organ donation for transplantation. The donor is treated systemically via intravenous infusion for at least 10 minute prior to the harvesting of the organ with a composition of the invention. The composition (Repair Composition 8) comprises a moiety the binds ICAM-1 as the anchor moiety and a moiety that binds CD90 as the hook moiety. The composition furthers comprises VEGF as a signaling factor. The composition comprises a biodegradable material to which hook, anchor and signaling factor moieties are bound.

The transplantation team harvests the organ using standard techniques and flushes the organ with a solution containing the repair composition. Optionally, prior to transplantation into the recipient, the organ is again flushed with the repair composition.

The clinician may treat the recipient at the time of transplantation or anytime thereafter in the first month with the repair composition, which can be administered local or distant to the site of organ transplantation. Subsequently, upon clinical signs of rejection of the transplant, the physician may administer a repair composition to the patient again. The repair composition may be the same as administered initially, or may be different.

### Experimental Example 15

Immunotherapy has long been based on the enhancement of the body's own immune response to cancers. In most cases the response is inadequate in the face of metastatic cancers, in particular, because the immune cells (such as helper T cells (CD4+), killer T cells (CD8+) and Natural Killer (NK) cells) need to be brought into close contact with the tumor antigens on the surface of the cells. There may be an insufficient response to the oncogenic protein. The cytokine environment does not allow amplification of helper T cells to occur, which results in low levels of cytokines. Antigen-presenting cells, which stimulate T cell activity, may also not be functional.

The following experiments are designed to identify compositions that bind immune cells to tumor cells.

Repair compositions are prepared as described previously, using biotinylated moieties and binding them to avidin. The hook moiety is selected from anti-rat CD3, anti-rat CD8 and anti-rat CD4. The anchor moiety is a molecule, such as an antibody, that binds to one of MAGE 1, 2, or 3, MART-1/Melan-A, gp100, carcinoembryonic antigen (CEA), HER-2, mucins (i.e., MUC-1), prostate-specific antigen (PSA), prostatic acid phosphatase (PAP), hepatitis B (HBV), Epstein-Barr (EBV), human papilloma (HPV), p53, or glycosylate proteins. Each repair composition may further comprise a cytokine, such as IL-2, IL-4, IL-5, or IL-13. Each repair composition may further comprise a molecule, such as an antibody, that binds to one of CDw137, B7-H2, CD275, CD28, CD40 (BP50), CD80 (B7-1), CD86 (B7-2), CD150 (SLAM), CD154 (CD40 Ligand), GITR Ligand, ICOS (CD278), or ICOSL (B7-H2, CD275).

Single cell suspensions of human tumor cell lines, such as MART-1 positive melanoma cells, are injected into the athymic tissue, such as heart (a common metastatic site for human melanoma) of athymic nude rats, and allowed to grow for 3 days. A control solution or a repair composition consisting of an anchor to melanoma, such as MART-1, hooks to markers on rat immunogenic cells, such as CD3 and/or costimulatory molecules, as well as stimulatory cytokines, which will amplify T cell responses, are delivered IV or locally to the animals. Spleen and bone marrow cells from euthymic rats are isolated and injected into the treated or control athymic nude rats.

Treated animals are evaluated in terms of survival, tumor growth and immune response, compared to non-treated, control rats. Repair compositions providing a therapeutic benefit in at least one aspect are deemed useful in the treatment of such cancers. As the skilled artisan will recognize, such repair compositions may be reformulated for administration to a human to treat a cancer. For instance, a repair composition may comprise an antibody to the human analog of a rat target, such as human CD3, human CD4 or human CD8.

### Experimental Example 16

Cancer vaccines, which target a single protein, may not supply the long-term immunity necessary to prevent cancer relapse. Therefore, there is a need to discover antigenic formulations that target multiple antigens. Clinically useful tumor vaccines will immunize against multiple immunogenic proteins.

The following experiments are designed to identify compositions that are active as multiple antigen cancer vaccines.

Repair compositions are prepared as described previously, using biotinylated moieties and binding them to avidin. The hook moiety is selected from anti-rat CD1a, anti-rat HLA Class 2, anti-rat CD80, anti-rat CD86, anti-rat CD40, anti-rat CD54, anti-rat CD11c, and anti-rat CD206 (mannose receptor). The anchor moiety is an human tumor antigen selected from MAGE 1, 2, or 3, MART-1/Melan-A, gp100, carcinoembryonic antigen (CEA), HER-2, mucins (i.e., MUC-1), prostate-specific antigen (PSA), prostatic acid phosphatase (PAP), hepatitis B (HBV), Epstein-Barr (EBV), human papilloma (HPV), p53, and glycosylate proteins. Each repair composition optionally comprises a cytokine, such as IL-2, IL-4, IFN-gamma, IL-12 and IL13. Each repair composition may further optionally comprise an antibody to a co-stimulatory molecule selected from CDw137, B7-H2, CD275, CD28, CD40 (BP50), CD80 (B7-1), CD86 (B7-2), CD150 (SLAM), CD154 (CD40 Ligand), GITR Ligand, ICOS (CD278), and ICOSL (B7-H2, CD275).

Single cell suspensions of a metastatic cell line, such as MAT-LyLu (a metastatic rat prostate cell line) are injected into an athymic rat and allowed to grow for 3 days. A control solution or a repair composition consisting of an tumor antigen for prostate cancer, such as PSA, as the anchor, a marker on rat antigen-presenting cells, such as dendritic cells, as the hook target, a molecule that binds a costimulatory molecule, and a stimulatory cytokine, which will enhance antigen presentation and immune response, is administered by either IV or local injection. Spleen and bone marrow cells from euthymic rats are isolated and injected into the treated or control athymic nude rats.

Treated animals are evaluated in terms of survival, tumor growth and immune response, compared to non-treated, control rats. Repair compositions providing a therapeutic benefit in at least one aspect are deemed useful as a cancer vaccine. As the skilled artisan will recognize, such repair compositions may be reformulated for administration to a human as a cancer vaccine. For instance, a repair composition may comprise an antibody to the human analog of a rat target, such as human HLA Class 2, human CD80, human CD86, human CD40, human CD54, human CD 11c or human CD206.

### Experimental Example 17

The bone marrow environment is dependent upon the interplay among multiple stem cells, stromal cells and recovery signals. After injury to the bone marrow (accidental or intentional, such as for treatment of cancer) the stem cell recovery is dependent on the timely interaction of all of these elements for bone marrow recovery. To date, there has been no successful means for targeting bone marrow and delivering multiple stimulatory signals.

Prior studies with anti-CD45 have shown good targeting to the bone marrow, as well as other stromal cell markers. In addition, it is known that in vivo, the biotin on biotinylated moieties may be cleaved off the moiety by the enzymatic action of biotinidase (a biotin-amide amidohydrolase). This cleavage occurs prior to the dissociation of biotin from avidin. Thus, a repair composition comprising biotinylated anti-CD45 antibody and two or more biotinylated signaling factors bound to avidin is expected to target the signaling factors to the bone marrow. Signaling factors, for instance those that initiate signaling by binding to an extracellular receptor, may function biologically while bound to avidin. Advantageously, their release, by cleavage of the biotin, will allow the signaling factors to also act on bystander cells in the bone marrow. Depending on the signaling factor, release from the biotin may required so that the factor is free to bind to and enter a cell. Erythropoietin (EPO) is an example of such a signaling factor.

The following experiments are designed to identify compositions that are useful for improving bone marrow recovery after injury, including, but not limited to, cancer chemotherapy, radiation injury, and chemical injury.

Repair compositions are prepared as previously described comprising biotinylated anti-mouse CD45 antibody and two or more biotinylated soluble factors, such as GM-CSF, SCF, and EPO.

Mice are irradiated according to a standard bone marrow ablation program. A control solution or a repair composition is administered by infusing intravenously or local infusion into the marrow cavity.

Treated animals are evaluated in terms of hematopoietic recovery and animal survival, compared to non-treated, control mice. Repair compositions providing a therapeutic benefit in at least one aspect are deemed useful as a therapeutic for aiding post bone marrow injury recovery. As the skilled artisan will recognize, such repair compositions may be reformulated for administration to a human to treat a bone marrow injury. For instance, a repair composition may comprise an antibody to the human analog of a mouse target, such as human CD45.

### Experimental Example 18

Human patients administered a repair composition of the invention would potentially be exposed systemically to the composition, even if administered locally. The following experiment was designed to characterize the in vivo safety and toxicity of a repair composition of the invention following intravenous administration of large doses to mice. The doses used were five times the dose administered to rat.

The materials and methods used in the experiments presented in this Example are now described.

Repair composition 2, comprising anti-CD54 antibody and anti-CD90 antibody, was prepared as follows. Twenty (20) µg biotinylated anti-CD54 antibody and 20 µg biotinylated anti-CD90 antibody (molar ratio 1:1) were mixed together, added to 20 µg avidin in a total volume of 1000 µl, and allowed to bind for 30 minutes at 22 °C. The repair composition solution was then diluted to a final volume of 2000 µl with PBS.

Healthy, adult male CD-1 mice, 30 - 40 grams, were obtained from a local supplier. Animals were acclimated to the laboratory conditions for a minimum of 1 week, fed a standard laboratory diet and provided with tap water ad libitum.

Per an IACCUC-approved protocol, 20 mice each were administered 100 µl of the repair composition on Study Day 1. Doses were administered as an IV bolus into one of the lateral tail veins. Following dosing, animals were observed daily for general health and welfare. Groups of 6 animals each were humanely euthanized on Days 3 and 7, and the remaining 8 mice were humanely euthanized on Day 10 following dose administration. At the time of euthanasia, animals were weighed, blood was collected for hematology and clinical chemistry determinations, and each animal was subjected to a gross necropsy. Major organs were observed, and abnormalities in gross anatomy were noted.

The results of the experiments in this example are now presented.

Clinical Observations: All animals appeared normal during dosing, within 1 hour of dosing, and during daily examinations for the 10 days following dosing. There were no significant clinical observations noted in any animals during the course of the study.

Body weights: All animals appeared to gain weight normally during the course of the study. -

Necropsy observations: Animal #13 (Day 10) was observed to have an enlarged spleen at the time of scheduled euthanasia. All other organs in all animals euthanized at 3, 7 and 10 days appeared normal.

A summary of the hematology and serum chemistry results is now presented. "Test article" refers to the repair composition administered.

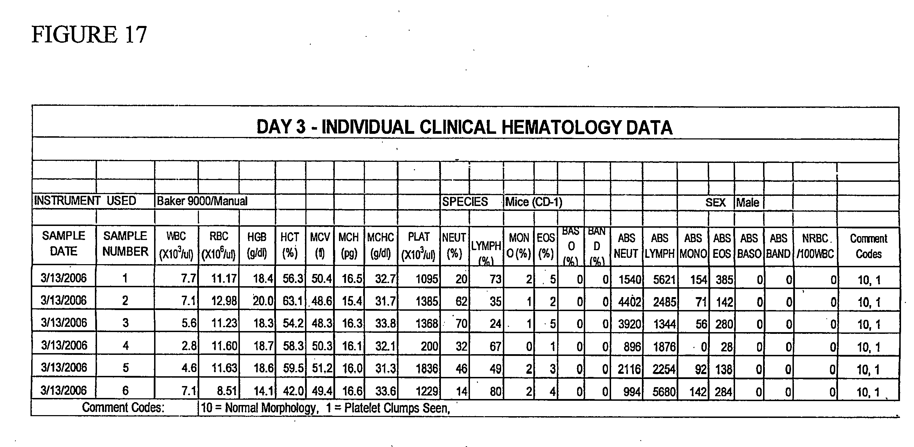

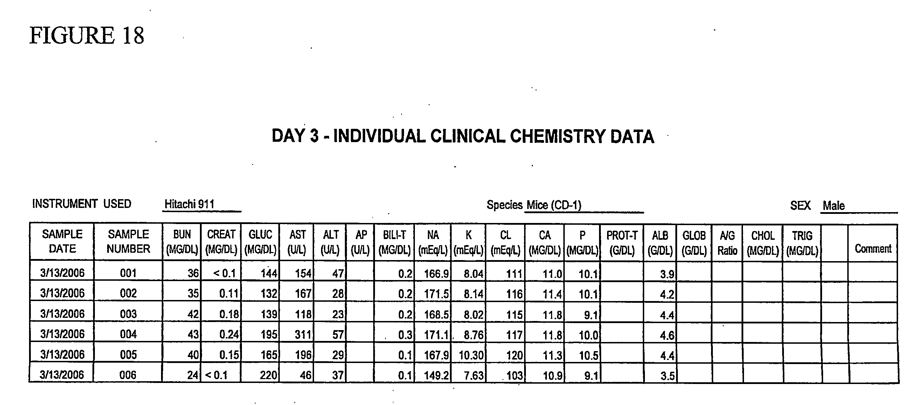

Day 3: No toxicologically-significant test article related effects were observed on hematology or chemistry parameters (Figures 17 and 18). One animal (#4) was observed to have lowered WBC counts (129% lower than group mean), platelets and neutrophils; these data correlated with increased AST (128% higher than group mean). No anatomic or clinical correlation was noted. These data for animal #4 are consistent with immunosuppression of unknown etiology (spurious).

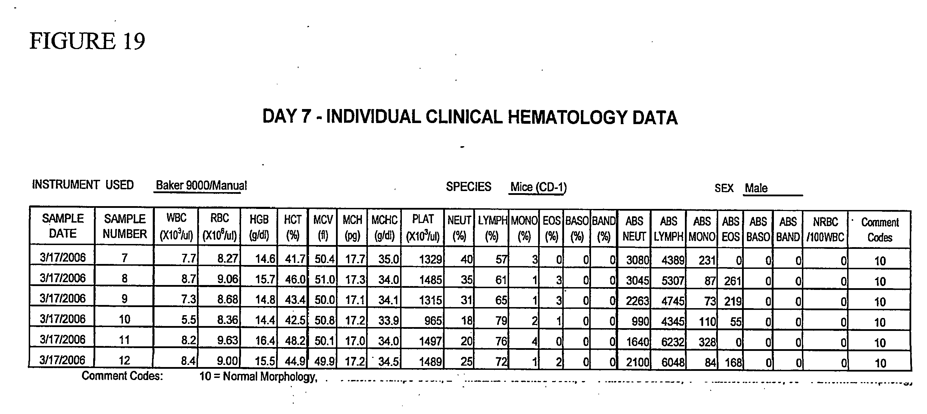

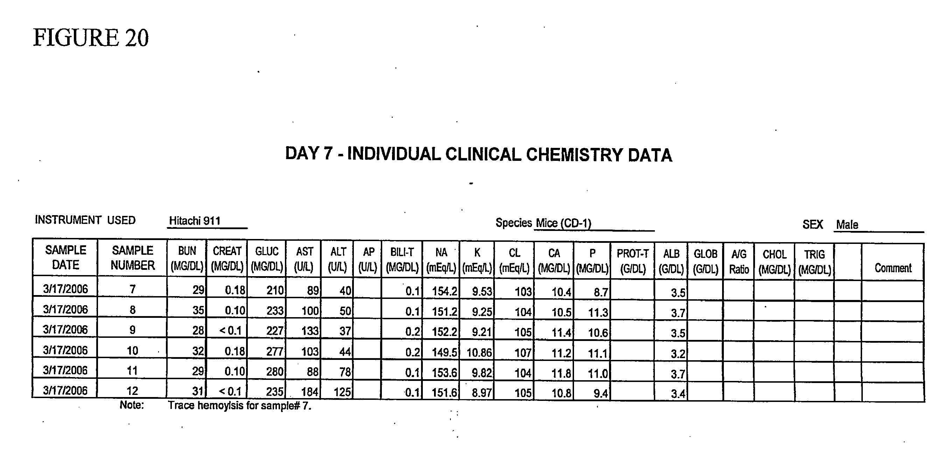

Day 7: No toxicologically-significant test article related effects were observed on hematology or chemistry parameters (Figures 19 and 20). One animal was observed to have decreased platelets and neutrophils (60% decrease) of unknown origin.

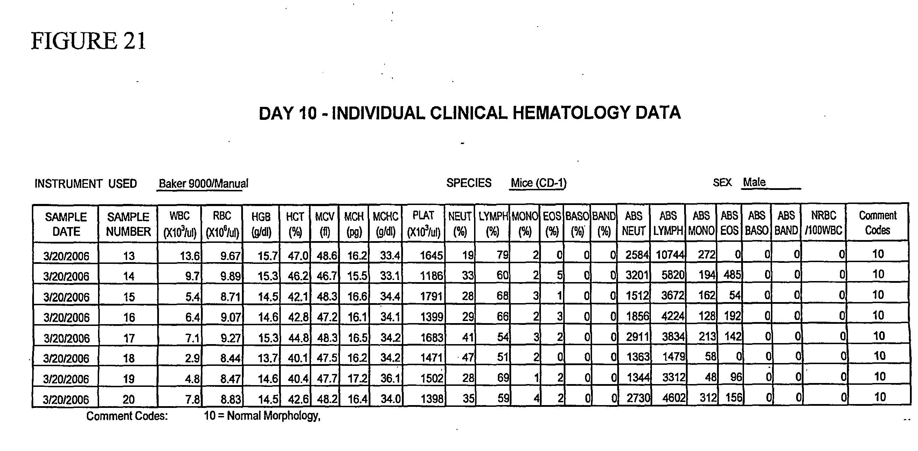

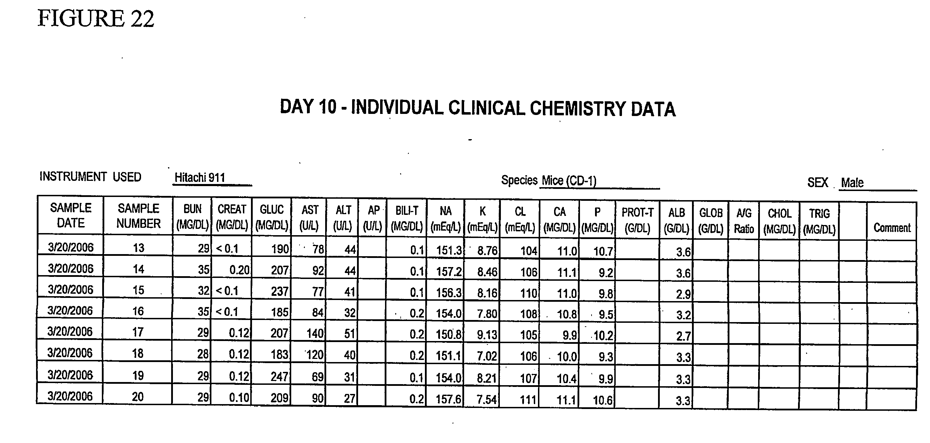

Day 10: No toxicologically-significant test article related effects were observed on hematology or chemistry parameters (Figures 21 and 22). One animal (#13) was observed with increased WBC (116% higher than the group mean) and markedly increased lymphocytes (179% increase over group mean). This animal had correlative splenomegally on gross necropsy.

These data indicate that IV administration of large doses of a repair composition of the invention to adult, male CD-1 mice did not result in obvious, deleterious effects.

The disclosures of each patent, patent application and publication cited herein are hereby incorporated herein by reference in their entirety.

While this invention has been disclosed with reference to specific embodiments, it is apparent that other embodiments and variations of this invention may be devised by those of skill in the art without departing from the spirit and scope of the invention.

### The invention provides the following items:

1. A tissue repair composition comprising: a first cell binding moiety that preferentially binds a damaged or defective cell; and a second cell binding moiety that preferentially binds a repair cell; wherein said first and second cell binding moieties are linked.
2. The tissue repair composition of item 1, wherein said first and second cell binding moieties are linked to a common substrate.
3. The tissue repair composition of item 1, wherein said second cell binding moiety preferentially binds a stem cell.
4. The tissue repair composition of item 1 , wherein said second cell binding moiety preferentially binds a cell surface molecule selected from the group comprising CD44, CD45, CD90, CD105, CD133, CD34, CD33, CD38, CD105, CD106, nestin and combinations thereof.
5. The tissue repair composition of item 4, wherein said first cell binding moiety preferentially binds an integrin.
6. The tissue repair composition of item 4, wherein said first cell binding moiety preferentially binds a cell surface molecule selected from the group comprising ICAM-I, N-CAM, a selectin, a cadherin, endothelin, CD44, V-CAM, P-CAM, collagen, fibronectin, hyaluronic acid, a proteoglycan, TGF-beta receptor and combinations thereof.
7. The tissue repair composition of item 1, wherein said repair cell is selected from the group comprising an embryonic stem cell, a multipotent adult stem cell and a stromal cell.
8. The tissue repair composition of item 7, wherein said stromal cell is a bone marrow stromal cell or an adipose tissue derived stromal cell.
9. The tissue repair composition of item 2, wherein said common substrate is avidin and at least one of said cell binding moieties is biotinylated.
10. The tissue repair composition of item 2, wherein said common substrate comprises a polymer.
11. The tissue repair composition of item 2, wherein said common substrate is a hydrogel.
12. The tissue repair composition of item 1 further comprising a signaling factor linked to said first and second cell binding moieties.
13. The tissue repair composition of item 12, wherein said signaling factor is selected from the group consisting of EGF, VEGF, NGF, FGF, EPO, G-CSF, GM-CSF, PDGF, IGF-I, stem cell factor, stromal derived factor (SDF-1), MMP inhibitors, LIF, flt-1 ligand and combinations thereof.
14. A tissue repair composition comprising: a first cell binding moiety that preferentially binds ICAM-I ; and a second cell binding moiety that preferentially binds CD90; wherein said first and second cell binding moieties are linked.
15. The tissue repair composition of item 14, wherein said first and second cell binding moieties are linked to a common substrate.
16. The tissue repair composition of item 15, wherein said common substrate is avidin and at least one of said cell binding moieties is biotinylated.
17. The tissue repair composition of item 14, wherein at least one of said cell binding moieties is an antibody.
18. The tissue repair composition of item 14, wherein said composition further comprises a signaling factor, wherein said signaling factor is selected from the group consisting of EGF, VEGF, NGF, FGF, EPO, G-CSF, PDGF, IGF-I, stem cell factor, stromal derived factor, MMP inhibitors, LIF and combinations thereof, and wherein said signaling factor is linked to said first and second cell binding moieties.
19. The tissue repair composition of item 18, wherein said signaling factor is EGF.
20. A pharmaceutical composition comprising a tissue repair composition and a pharmaceutically acceptable excipient, wherein said tissue repair composition comprises a first cell binding moiety that preferentially binds a damaged or defective cell; and a second cell binding moiety that preferentially binds a repair cell; wherein said first and second cell binding moieties are linked.
21. The pharmaceutical composition of item 20, wherein said composition is a solution.
22. The pharmaceutical composition of item 20, wherein said composition is bound to a matrix.
23. A pharmaceutical composition comprising a tissue repair composition and a pharmaceutically acceptable excipient, wherein said tissue repair composition comprises a first cell binding moiety that preferentially binds ICAM-I; and a second cell binding moiety that preferentially binds CD90; wherein said first and second cell binding moieties are linked.
24. A method of improving repair of injured tissue, said method comprising contacting injured tissue with a therapeutically effective amount of a tissue repair composition, wherein said tissue composition comprises: a first cell binding moiety that preferentially binds a damaged or defective cell of said injured tissue; and a second cell binding moiety that preferentially binds a repair cell; wherein said first and second cell binding moieties are linked.
25. A method of treating a mammal that has suffered an ischemic injury, said method comprising administering a therapeutically effective amount of a tissue repair composition to said mammal, wherein said tissue repair composition comprises: a first cell binding moiety that preferentially binds a cell that is damaged or defective due to the ischemic injury; and a second cell binding moiety that preferentially binds a repair cell; wherein said first and second cell binding moieties are linked.
26. The method of item 25, wherein said mammal is a human.
27. A method of reducing tissue damage in a mammal that has suffered a stroke, said method comprising administering a therapeutically effective amount of a tissue repair composition to said mammal, wherein said tissue repair composition comprises: a first cell binding moiety that preferentially binds a cell that is damaged or defective due to the stroke; and a second cell binding moiety that preferentially binds a repair cell; wherein said first and second cell binding moieties are linked.
28. The method of item 27, wherein said mammal is a human.
29. A method of enhancing tissue repair in a mammal that has suffered a myocardial infarction, said method comprising administering a therapeutically effective amount of a tissue repair composition to said mammal, wherein said tissue repair composition comprises: a first cell binding moiety that preferentially binds a cell that is damaged or defective due to the myocardial infarction; and a second cell binding moiety that preferentially binds a repair cell; wherein said first and second cell binding moieties are linked.
30. The method of item 29, wherein said mammal is a human.
31. A method of enhancing tissue repair in a mammal that has suffered a spinal cord injury, said method comprising administering a therapeutically effective amount of a tissue repair composition to said mammal, wherein said tissue repair composition comprises: a first cell binding moiety that preferentially binds a cell that is damaged or defective due to the spinal cord injury; and a second cell binding moiety that preferentially binds a repair cell; wherein said first and second cell binding moieties are linked.
32. The method of item 31, wherein said mammal is a human.
33. A method of diminishing the severity of reperfusion injury in a mammal, said method comprising administering a therapeutically effective amount of a tissue repair composition to said mammal, wherein said tissue repair composition comprises: a first cell binding moiety that preferentially binds a cell that is damaged or defective due to the reperfusion injury; and a second cell binding moiety that preferentially binds a repair cell; wherein said first and second cell binding moieties are linked.
34. The method of item 33 wherein said mammal has received an organ transplant.
35. The method of item 34, wherein said organ for said organ transplant is chosen from the group comprising kidney, heart, lung, liver, pancreas, blood vessel, retina, skin and combinations thereof.
36. The method of item 33, wherein said reperfusion injury occurs upon reperfusion after vascular stenting, upon reperfusion after vascular angioplasty, upon reperfusion after thrombolytic therapy, upon reperfusion after coronary artery bypass surgery, or upon reperfusion after intestinal surgery.
37. A method of diminishing the severity of reperfusion injury to an organ intended for transplant, said method comprising contacting said organ with a therapeutically effective amount of a tissue repair composition, wherein said tissue repair composition comprises: a first cell binding moiety that preferentially binds a cell that is damaged or defective due to the reperfusion injury; and a second cell binding moiety that preferentially binds a repair cell; wherein said first and second cell binding moieties are linked.
38. A method of enhancing tissue repair in damaged cartilage in a mammal, said method comprising administering a therapeutically effective amount of a tissue repair composition to said mammal, wherein said tissue repair composition comprises: a first cell binding moiety that preferentially binds a damaged or defective cell in said damaged cartilage; and a second cell binding moiety that preferentially binds a repair cell; wherein said first and second cell binding moieties are linked.
39. A method of enhancing tissue repair in damaged skin in a mammal, said method comprising administering a therapeutically effective amount of a tissue repair composition to said mammal, wherein said tissue repair composition comprises: a first cell binding moiety that preferentially binds a damaged or defective cell in said damaged skin; and a second cell binding moiety that preferentially binds a repair cell; wherein said first and second cell binding moieties are linked.
40. A method of treating renal damage in a mammal, said method comprising administering a therapeutically effective amount of a tissue repair composition to said mammal, wherein said tissue repair composition comprises: a first cell binding moiety that preferentially binds a damaged or defective renal cell; and a second cell binding moiety that preferentially binds a repair cell; wherein said first and second cell binding moieties are linked.
41. The method according to any of items 33-36 and 38-40, wherein said mammal is a human.
42. The method according to any of items 24-40, wherein said first and second cell binding moieties are linked to a common substrate.
43. The method according to any of items 24-40, wherein said common substrate is one of avidin, a polymer and a hydrogel.
44. The method according to any of items 24-40, further comprising a signaling factor linked to said first and second cell binding moieties.

## Claims

1. A tissue repair composition comprising: a first cell binding moiety that preferentially binds CD 105; and a second cell binding moiety that preferentially binds CD90; wherein said first and second cell binding moieties are linked.

2. The tissue repair composition of claim 1, wherein said first and second cell binding moieties are linked to a common substrate.

3. The tissue repair composition of claim 2, wherein said common substrate is avidin and at least one of said cell binding moieties is biotinylated.

4. The tissue repair composition of claim 2, wherein said common substrate comprises a polymer.

5. The tissue repair composition of claim 2, wherein said common substrate is a hydrogel.

6. The tissue repair composition of any one of claims 1 to 5, wherein at least one of said cell binding moieties is an antibody.

7. The tissue repair composition of claim 1, wherein said composition further comprises a signaling factor, wherein said signaling factor is selected from the group consisting of EGF, VEGF, NGF, FGF, EPO, G-CSF, PDGF, IGF-1, stem cell factor, stromal derived factor, MMP Inhibitors, LIF, and combinations thereof, and wherein said signaling factor is linked to said first and second cell binding moieties.

8. The tissue repair composition of claim 7, wherein said signaling factor is EGF.

9. A pharmaceutical composition comprising a tissue repair composition of any one of claims 1 to 8 and a pharmaceutically acceptable excipient.

10. A tissue repair composition of any one of claims 1 to 8 for use in:
(a) enhancing tissue repair in a mammal;
(b) improving repair of injured tissue;
(c) treating renal damage in a mammal; or
(d) reducing tissue damage in a mammal.

11. Use of the tissue repair composition of any one of claims 1 to 8 for the preparation of a pharmaceutical composition for:
(a) enhancing tissue repair in a mammal;
(b) improving repair of injured tissue;
(c) treating renal damage in a mammal; or
(d) reducing tissue damage in a mammal.

12. The use according to claim 11, wherein said mammal is a human.

13. The use according to claim 11 or 12, wherein the first and second cell binding moieties are linked to a common substrate.

14. The use according to any one of claims 11 to 13, wherein said common substrate is one of avidin, a polymer or a hydrogel.

15. A tissue repair composition of any of claims 1 to 8 for use in therapy or prophylaxis or diagnosis.
